# EUROPÄISCHE PATENTSCHRIFT

(11) **EP 1 185 505 B1**
(45) Veröffentlichungstag und Bekanntmachung des Hinweises auf die Patenterteilung: **03.11.2004**
(21) Anmeldenummer: 00929527.0
(22) Anmeldetag: 12.05.2000
(51) Int. Cl.: C07C 249/00, C07C 251/48, C07D 239/34

(54) **VERFAHREN ZUR HERSTELLUNG VON METHOXYIMINO-ESSIGSÄUREAMIDEN**
METHOD OF PRODUCING METHOXYIMINO ACETIC AMIDE
PROCEDES DE PREPARATION D'AMIDES D'ACIDE ACETIQUE METHOXYIMINO

(30) Priorität: 25.05.1999 DE 19923624; 11.01.2000 DE 10000733
(43) Veröffentlichungstag der Anmeldung: 13.03.2002
(73) Patentinhaber: Bayer CropScience AG, 40789 Monheim (DE)
(72) Erfinder: GAYER, Herbert, D-40789 Monheim (DE); GALLENKAMP, Bernd, D-42133 Wuppertal (DE); GERDES, Peter, D-52080 Aachen (DE); HEINEMANN, Ulrich, D-42799 Leichlingen (DE); HÜBSCH, Walter, D-42113 Wuppertal (DE); KRÜGER, Bernd-Wieland, D-51467 Bergisch Gladbach (DE); MAURER, Fritz, D-40789 Monheim (DE); WEINTRITT, Holger, D-40764 Langenfeld (DE)
(86) Internationale Anmeldenummer: PCT/EP2000/004300
(87) Internationale Veröffentlichungsnummer: WO 2000/071504

(56) Entgegenhaltungen:
- EP-A- 0 398 692
- EP-A- 0 846 691
- DE-A- 19 525 969
- DE-A- 19 646 407
- DE-A- 19 706 396
- DE-A- 19 706 399
- GB-A- 2 253 624

## Beschreibung

Die vorliegende Erfindung betrifft neue Verfahren zur Herstellung von Methoxyimino-essigsäureamiden.

Ein Verfahren zur Herstellung von *N*-Methyl-[2-(2-hydroxy)phenyl]-2-methoxyimino-essigsäureamid wurde bereits beschrieben (vgl. EP 0398 692 A2). Die nach diesen Verfahren hergestellten Verbindungen sind jedoch nur in mäßigen Ausbeuten erhältlich.

Es wurde nun gefunden, dass man nach Verfahren Teil 1) Verbindungen der Formel (I), 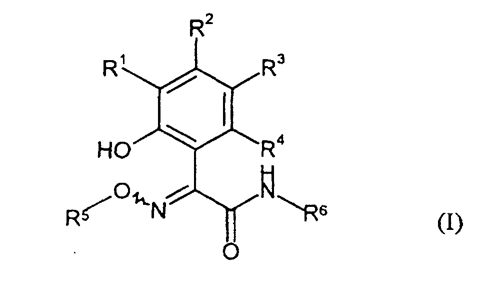 in welcher
- R¹, R², R³ und R⁴: gleich oder verschieden sind und unabhängig voneinander jeweils für Wasserstoff, Halogen, Cyano, Nitro, jeweils gegebenenfalls durch Halogen substituiertes Alkyl, Alkoxy, Alkylthio. Alkylsulfinyl oder Alkylsulfonyl stehen,
- R⁵: für substituiertes oder unsubstuiertes Alkyl steht.
- R⁶: für Wasserstoff, substituiertes oder unsubstituiertes Alkyl steht,
erhält,
A) dadurch, dass man nach Verfahrensschritt 2) Verbindungen der Formel (IV), 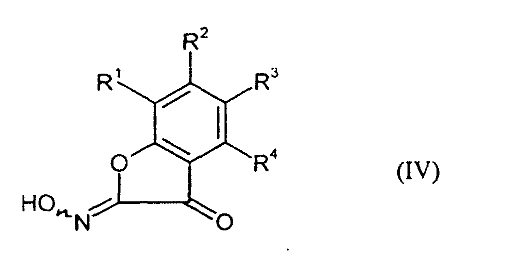 in welcher
   - R¹, R², R³ und R⁴: die oben angegebenen Bedeutungen haben,
   in Gegenwart einer Säure oder eines sauren Ionenaustauschers mit einem Alkohol der Formel (V),

   R⁷-OH (V)

   in welcher
   - R⁷: für substituiertes oder unsubstituiertes Alkyl steht,
   und mit einer Carbonylverbindung, die das bei der Reaktion abgespaltene Hydroxylammoniumchlorid unter Oximbildung bindet, zu Verbindungen der Formel (VI) umsetzt, 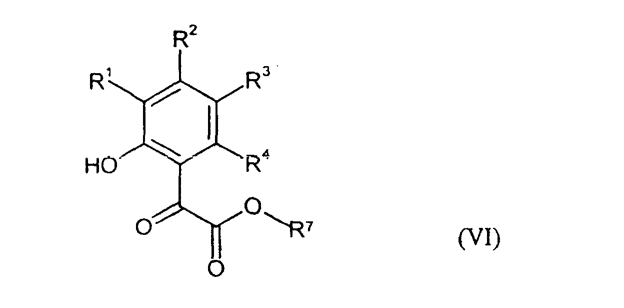 in welcher
   - R¹, R², R³, R⁴ und R⁷: die oben angegebenen Bedeutungen haben,
   und die so erhaltenen Verbindungen der Formel (VI) entweder
   a) nach Verfahrensschritt 3) mit einem Hydroxylammoniumsalz gegebenenfalls in Gegenwart eines Verdünnungsmittels und gegebenenfalls in Gegenwart einer Säure oder eines Säureakzeptors zu Verbindungen der Formel (VII). 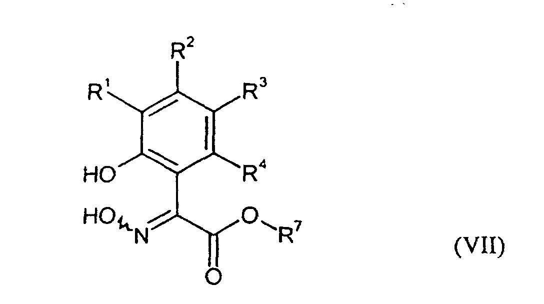 in welcher
      - R¹, R², R³, R⁴ und R⁷: die oben angegebenen Bedeutungen haben,
      umsetzt, und die so erhaltenen Verbindungen der Formel (VII) nach Verfahrensschritt 4) mit einem Alkylierungsmittel der Formel (VIII),

      R⁵-X (VIII)

      in welcher
      - R⁵: die oben angegebene Bedeutung hat, und
      - X: für Halogen, -O-CO-OR⁵- oder -O-SO₂-O-R⁵ steht, wobei R⁵ die oben angegebene Bedeutung hat,
      gegebenenfalls in Gegenwart eines Verdünnungsmittels und gegebenenfalls in Gegenwart einer Base umsetzt,
      oder
   b) nach Verfahrenschritt 5) mit einem Alkoxyamin der Formel (IX),

      R⁵-O-NH₂ (IX)

      in welcher
      - R⁵: die oben angegebene Bedeutung hat,
      - oder einem Säureadditionskomplex davon - gegebenenfalls in Gegenwart eines Verdünnungsmittels und gegebenenfalls in Gegenwart einer Säure oder eines Säureakzeptors umsetzt,
   oder
B ) dadurch, dass man nach Verfahrensschritt 6) Verbindungen der Formel (IV), 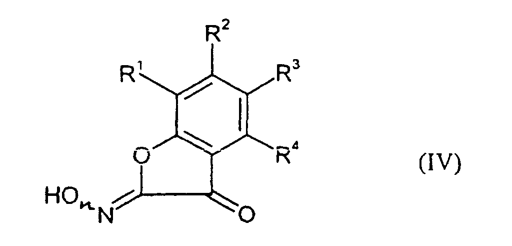 in welcher
   - R¹, R², R³ und R⁴: die oben angegebenen Bedeutungen haben,
   mit einem Alkoxyamin der Formel (IX),

   R⁵-O-NH₂ (IX)

   in welcher
   - R⁵: die oben angegebene Bedeutung hat,
   -oder einem Säureadditionskomplex davon- gegebenenfalls in Gegenwart eines Verdünnungsmittels und gegebenenfalls in Gegenwart einer Säure umsetzt,
   oder
C) dadurch, dass man nach Verfahrensschritt 7) Verbindungen der Formel (IV), 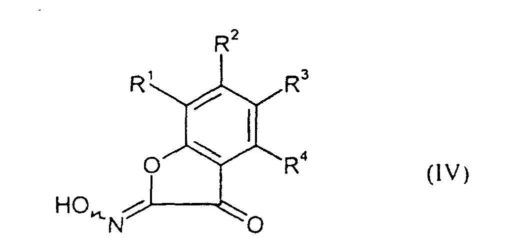 in welcher
   - R¹, R², R³ und R⁴: die oben angegebenen Bedeutungen haben,
   in Gegenwart einer Säure oder eines sauren Ionenaustauschers mit einem Alkohol der Formel (V),

   R⁷-OH (V)

   in welcher R⁷ die oben angegebene Bedeutung hat, gegebenenfalls unter Zusatz eines Hydroxylammoniumsalzes umsetzt, und die so erhaltenen Verbindungen der Formel (VII), 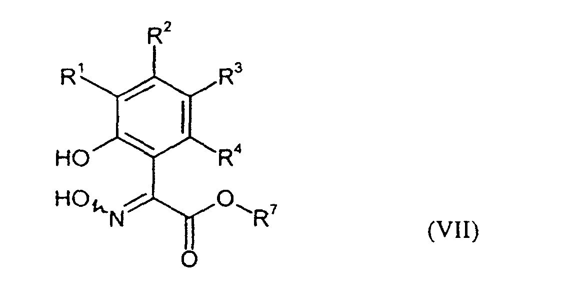 in welcher
   - R¹, R², R³, R⁴ und R⁷: die oben angegebenen Bedeutungen haben,
   nach Verfahrensschritt 4) umsetzt,
   oder
D) dadurch, dass man nach Verfahrensschritt 8) Verbindungen der Formel (X), 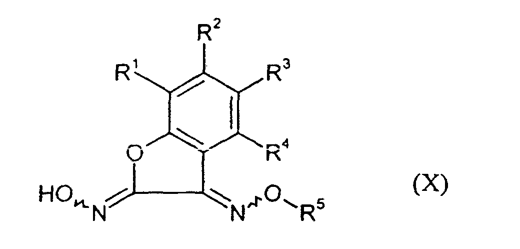 in welcher
   - R¹, R², R³, R⁴ und R⁵: die oben angegebenen Bedeutungen haben,
   in Gegenwart einer Säure oder eines sauren Ionenaustauschers mit einem Alkohol der Formel (V),

   R⁷-OH (V)

   in welcher R⁷ die oben angegebene Bedeutung hat, gegebenenfalls in Anwesenheit einer Carbonylverbindung, die das bei der Reaktion abgespaltene Hydroxylammoniumchlorid unter Oximbildung bindet, umsetzt,
   und die so nach den Verfahren A)-D) erhaltenen Verbindungen der Formel (II), 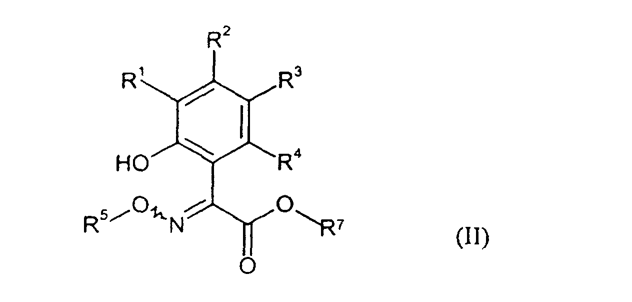 in welcher
   - R¹, R², R³, R⁴ und R⁵: die oben angegebenen Bedeutungen haben, und
   - R⁷: für unsubstituiertes oder substituiertes Alkyl steht.
   gegebenenfalls ohne Zwischenisolierung der Verbindungen der Formel (II) (Eintopfverfahren),
   nach Verfahrensschritt 1) mit einem Alkylamin der Formel (III),

   R⁶-NH₂ (III)

   in weicher R⁶ die oben angegebene Bedeutung hat, gegebenenfalls in Gegenwart eines Verdünnungsmittels umsetzt.
   Außerdem wurde gefunden, dass man nach Verfahren Teil 2) Verbindungen der Formel (XI), 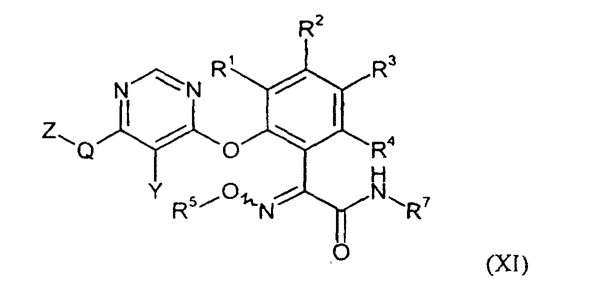 in welcher
   - Z: für unsubstituiertes oder substituiertes Cycloalkyl, Aryl oder Heterocyclyl steht,
   - Q: für Sauerstoff oder Schwefel steht,
   - Y: für Halogen steht und
   - R¹, R², R³, R⁴, R⁵ und R⁷: die oben angegebenen Bedeutungen haben,
   erhält, wenn man Verbindungen der Formel (I) nach dem neuen Verfahren Teil 1) umsetzt, und diese Verbindungen (I) entweder
E) nach Verfahrensschritt 9) mit Pyrimidin-Derivaten der Formel (XII), 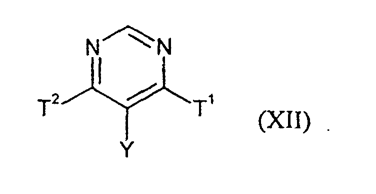 in welcher
   - T¹ und T²: gleich oder verschieden sind und für Halogen oder -SO₂-R⁸ stehen, wobei R⁸ für Alkyl, Aryl oder Benzyl steht, und
   - Y: die oben angegebene Bedeutung hat,
   gegebenenfalls in Gegenwart eines Verdünnungsmittels und gegebenenfalls in Gegenwart einer Base umsetzt, und die so erhaltenen Verbindungen der Formel (XIII), 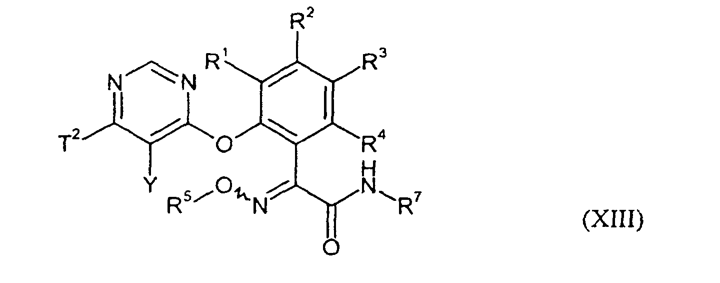 in welcher
   - T²,Y, R¹, R², R³, R⁴,R⁵ und R⁷: die oben angegebenen Bedeutungen haben,
   nach Verfahrensschritt 10) mit einer Ringverbindung der allgemeinen Formel (XIV),

   Z-Q-H (XIV)

   in welcher
   Z und Q die oben angegebenen Bedeutungen haben,
   gegebenenfalls in Gegenwart eines Verdünnungsmittels und gegebenenfalls in Gegenwart eines Säureakzeptors und gegebenenfalls in Gegenwart eines Katalysators umsetzt,
   oder
F) nach Verfahrensschritt 11) mit Verbindungen der Formel (XV), 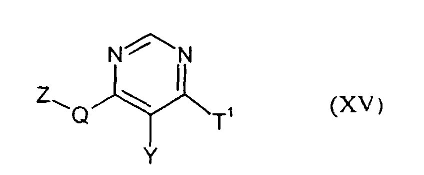
in welcher
- Z, Q, T¹ und Y: die oben angegebenen Bedeutungen haben,
gegebenenfalls in Gegenwart eines Verdünnungsmittels und gegebenenfalls in Gegenwart einer Base umsetzt.

Weiter wurde gefunden, dass die Z-isomeren Verbindungen der Formel (XI) zu E-isomeren Verbindungen der Formel (XI) isomerisiert werden, wenn man Z-Isomere oder E/Z-Isomerengemische der Verbindungen der Formel (XI) mit Säuren gegebenenfalls in einem Verdünnungsmittel behandelt. Durch die Isomerisierung werden die E-Isomeren in guten Ausbeuten erhalten.

Weiter wurde gefunden, dass die Z-isomeren Verbindungen der Formel (XIII) zu E-isomeren Verbindungen der Formel (XIII) isomerisiert werden, wenn man Z-Isomere oder E/Z-Isomerengemische der Verbindungen der Formel (XIII) mit Säuren gegebenenfalls in einem Verdünnungsmittel behandelt. Durch die Isomerisierung werden die E-Isomeren in guten Ausbeuten erhalten.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie beispielsweise in Alkoxy, Alkylthio oder Alkylamino, jeweils geradkettig oder verzweigt mit insbesondere 4 Kohlenstoffatomen.

Aryl steht für aromatische, mono- oder polycyclische Kohlenwasserstoffringe, wie z.B. Phenyl, Naphthyl, Anthranyl, Phenanthryl, vorzugsweise Phenyl oder Naphthyl, insbesondere Phenyl.

Halogen steht im allgemeinen für Fluor, Chlor, Brom oder Iod. vorzugsweise für Fluor, Chlor oder Brom, insbesondere für Fluor oder Chlor.

Heterocyclyl steht für gesättigte oder ungesättigte, sowie aromatische, ringförmige Verbindungen, in denen mindestens ein Ringglied ein Heteroatom. d. h. ein von Kohlenstoff verschiedenes Atom, ist. Enthält der Ring mehrere Heteroatome, können diese gleich oder verschieden sein. Heteroatome sind bevorzugt Sauerstoff, Stickstoff oder Schwefel. Gegebenenfalls bilden die ringförmigen Verbindungen mit weiteren carbocyclischen oder heterocyclischen, ankondensierten oder überbrückten Ringen gemeinsam ein polycyclisches Ringsystem. Bevorzugt sind mono- oder bicyclische Ringsysteme, insbesondere mono- oder bicyclische, aromatische Ringsysteme.

Cycloalkyl steht für gesättigte, carbocyclische Verbindungen, die gegebenenfalls mit weiteren carbocyclischen, ankondensierten oder überbrückten Ringen ein polycyclisches Ringsystem bilden.

Halogenalkyl steht für teilweise oder vollständig halogeniertes Alkyl. Bei mehrfach halogeniertem Halogenalkyl können die Halogenatome gleich oder verschieden sein. Bevorzugte Halogenatome sind Fluor und Chlor und insbesondere Fluor. Trägt das Halogenalkyl noch weitere Substituenten, reduziert sich die maximal mögliche Zahl der Halogenatome auf die verbleibenden freien Valenzen.

Die erfindungsgemäßen Verbindungen können gegebenenfalls als Mischungen verschiedener möglicher isomerer Formen, insbesondere von Stereoisomeren, wie z. B. E- und Z-, vorliegen. Es werden sowohl die E- als auch die Z-Isomeren, wie auch beliebige Mischungen dieser Isomeren, beansprucht.

Im allgemeinen steht Z insbesondere für:
jeweils gegebenenfalls einfach bis zweifach durch Halogen, Alkyl, oder Hydroxy substituiertes Cycloalkyl mit 3 bis 7 Kohlenstoffatomen;
für gegebenenfalls durch Alkyl mit 1 bis 4 Kohlenstoffatomen substituiertes Heterocyclyl mit 3 bis 7 Ringgliedern;
oder für jeweils gegebenenfalls einfach bis vierfach, gleich oder verschieden substituiertes Phenyl oder Naphthyl steht, wobei die möglichen Substituenten vorzugsweise aus der nachstehenden Aufzählung ausgewählt sind:
   Halogen, Cyano, Nitro, Amino, Hydroxy, Formyl, Carboxy, Carbamoyl, Thiocarbamoyl;
jeweils geradkettiges oder verzweigtes Alkyl, Hydroxyalkyl, Oxoalkyl, Alkoxy, Alkoxyalkyl, Alkylthioalkyl, Dialkoxyalkyl, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylaminocarbonyl, Dialkylaminocarbonyl, Arylalkylaminocarbonyl, Dialkylaminocarbonyloxy, Alkenylcarbonyl oder Alkinylcarbonyl, mit 1 bis 6 Kohlenstoffatomen in den jeweiligen Kohlenwasserstoffketten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Oxo, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen;
   oder eine Gruppierung 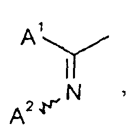 worin

- A¹: für Wasserstoff, Hydroxy oder Alkyl mit 1 bis 4 Kohlenstoffatomen oder Cycloalkyl mit 1 bis 6 Kohlenstoffatomen steht und
- A²: für Hydroxy, Amino, Methylamino, Phenyl, Benzyl oder für jeweils gegebenenfalls durch Cyano, Hydroxy, Alkoxy, Alkylthio, Alkylamino, Dialkylamino oder Phenyl substituiertes Alkyl oder Alkoxy mit 1 bis 4 Kohlenstoff atomen, oder für Alkenyloxy oder Alkinyloxy mit jeweils 2 bis 4 Kohlenstoffatomen steht,
sowie jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Phenoxy, Phenylthio, Benzoyl, Benzoylethenyl, Cinnamoyl, Heterocyclyl oder Phenylalkyl, Phenylalkyloxy, Phenylalkylthio, oder Heterocyclylalkyl, mit jeweils 1 bis 3 Kohlenstoffatomen in den jeweiligen Alkylteilen.

Im allgemeinen steht R⁵ insbesondere für Methyl oder Ethyl.

Im allgemeinen steht R⁶ insbesondere für Wasserstoff oder Methyl.

Im allgemeinen steht R⁷ insbesondere für Methyl.

Im allgemeinen steht Q insbesondere für Sauerstoff oder Schwefel.

Im allgemeinen steht Y insbesondere für Fluor, Chlor, Brom oder Iod.

Im allgemeinen steht T¹ insbesondere für Fluor oder Chlor.

Im allgemeinen steht T² insbesondere für Fluor oder Chlor.

Im allgemeinen sind R¹, R², R³ und R⁴ gleich oder verschieden und stehen unabhängig voneinander insbesondere jeweils für Wasserstoff, Halogen, Cyano, Nitro, jeweils gegebenenfalls durch 1 bis 5 Halogenatome substituiertes Alkyl Alkoxy, Alkylthio, Alkylsulfinyl oder Alkylsulfonyl mit jeweils 1 bis 6 Kohlenstoffatomen.

Bevorzugt sind Erfindungen, in denen Z für jeweils gegebenenfalls einfach bis zweifach durch Fluor, Chlor, Methyl, Ethyl oder Hydroxy substituiertes Cyclopentyl oder Cyclohexyl;
für gegebenenfalls durch Methyl oder Ethyl substituiertes Thienyl, Pyridyl oder Furyl;
oder für jeweils gegebenenfalls einfach bis vierfach, gleich oder verschieden substituiertes Phenyl oder Naphthyl steht, wobei die möglichen Substituenten vorzugsweise aus der nachstehenden Aufzählung ausgewählt sind:
Fluor, Chlor, Brom, Iod, Cyano, Nitro, Amino, Hydroxy, Formyl, Carboxy, Carbamoyl, Thiocarbamoyl
Methyl, Ethyl, n- oder 1-Propyl, n-, i-, s- oder t-Butyl, 1-, 2-, 3-, neo-Pentyl, 1-, 2-, 3-, 4-(2-Methylbutyl), 1-, 2-, 3-Hexyl, 1-, 2-, 3-, 4-, 5-(2-Methylpentyl), 1-,2-, 3-(3-Methylpentyl), 2-Ethylbutyl, 1-, 3-, 4-(2,2-Dimetylbutyl), 1-, 2-(2.3-Dimethylbutyl), Hydroxymethyl, Hydroxyethyl, 3-Oxobutyl, Methoxymethyl, Dimethoxymethyl, Methoxy, Ethoxy, n- oder i-Propoxy,
Methylthio, Ethylthio, n- oder i-Propylthio, Methylsulfinyl, Ethylsulfinyl, Methylsulfonyl oder Ethylsulfonyl,
Vinyl, Allyl, 2-Methylallyl, Propen-1-yl, Crotonyl, Propargyl, Vinyloxy, Allyloxy, 2-Methylallyloxy, Propen-1-yloxy, Crotonyloxy, Propargyloxy;
Trifluormethyl, Trifluorethyl,
Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl,
Methylamino, Ethylamino, n- oder i-Propylamino, Dimethylamino, Diethylamino, Acetyl, Propionyl, Methoxycarbonyl, Ethoxycarbonyl, Methylaminocarbonyl, Ethylaminocarbonyl, Dimethylaminocarbonyl, Diethylaminocarbonyl, Dimethylaminocarbonyloxy, Diethylaminocarbonyloxy, Benzylaminocarbonyl, Acryloyl, Propioloyl,
Cyclopentyl, Cyclohexyl,
jeweils gegebenenfalls einfach bis vierfach, gleich oder verschieden durch Fluor, Chlor, Oxo, Methyl oder Trifluormethyl substituiertes, jeweils zweifach verknüpftes Propandiyl, Ethylenoxy, Methylendioxy, Ethylendioxy
   oder eine Gruppierung 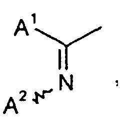 wobei
   - A¹: für Wasserstoff, Methyl oder Hydroxy steht und
   - A²: für Hydroxy, Methoxy, Ethoxy, Amino, Methylamino, Phenyl, Benzyl oder Hydroxyethyl steht, sowie
jeweils gegebenenfalls im Ringteil einfach bis dreifach durch Halogen, und/oder geradkettiges oder verzweigtes Alkyl oder Alkoxy mit 1 bis 4 Kohlenstoffatomen substituiertes Phenyl, Phenoxy, Phenylthio, Benzoyl, Benzoylethenyl, Cinnamoyl, Benzyl, Phenylethyl, Phenylpropyl, Benzyloxy, Benzylthio, 5,6-Dihydro-1,4,2-dioxazin-3-ylmethyl, Triazolylmethyl, Benzoxazol-2-ylmethyl, 1,3-Dioxan-2-yl, Benzimidazol-2-yl, Dioxol-2-yl, Oxadiazotyl.

Bevorzugt sind Verbindungen, in denen R⁵ für Methyl steht.

Bevorzugt sind Verbindungen, in denen R⁶ für Wasserstoff oder insbesondere Methyl steht.

Bevorzugt sind Verbindungen, in denen R⁷ für Methyl steht.

Bevorzugt sind Verbindungen, in denen Q für Schwefel oder insbesondere Sauerstoff steht.

Bevorzugt sind Verbindungen, in denen Y für Fluor oder Chlor steht.

Bevorzugt sind Verbindungen, in denen R¹, R², R³ und R⁴ gleich oder verschieden sind und unabhängig voneinander jeweils für Wasserstoff, Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methylsulfinyl, Ethylsulfinyl, Methylsulfonyl oder Ethylsulfonyl. Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl stehen.

In einer ganz besonders bevorzugten Gruppe von Verbindungen steht Z für gegebenenfalls substituiertes Phenyl.

In einer weiteren ganz besonders bevorzugten Gruppe von Verbindungen stehen
- R¹ und R³: unabhängig voneinander für Methyl und insbesondere Wasserstoff
und
- R² und R⁴: für Wasserstoff.

Besonders bevorzugt sind Verbindungen, in denen Y für Fluor steht.

Besonders bevorzugt sind Verbindungen, in denen Q für Sauerstoff steht.

Die oben aufgeführten allgemeinen oder in Vorzugsbereichen angegebenen Restedefinitionen gelten sowohl für die Endprodukte der Formel (I) und/oder der Formel (XI) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzelnen für diese Reste angegebenen Restedefinitionen werden unabhängig von der jeweilig angegebenen Kombination der Reste, beliebig auch durch Restedefinitionen anderer Vorzugsbereiche ersetzt.

Diese Restedefinitionen können untereinander, also auch zwischen den angegebenen Bereichen bevorzugter Verbindungen, beliebig kombiniert werden.

Die Verbindung der Formel (XI-1, E-Isomer) ist neu und erfinderisch und ebenfalls Gegenstand der Erfindung. Sie kann beispielsweise als Schädlingsbekämpfungsmittel verwendet werden.

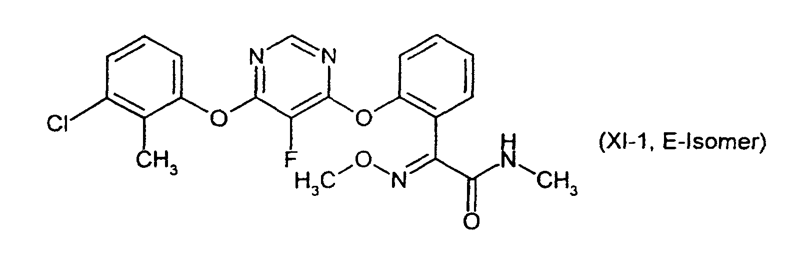

Die Verbindung der Formel (XI-1, Z-Isomer) ist neu und erfinderisch und ebenfalls Gegenstand der Erfindung. Sie kann beispielsweise als Schädlingsbekämpfungsmittel verwendet werden.

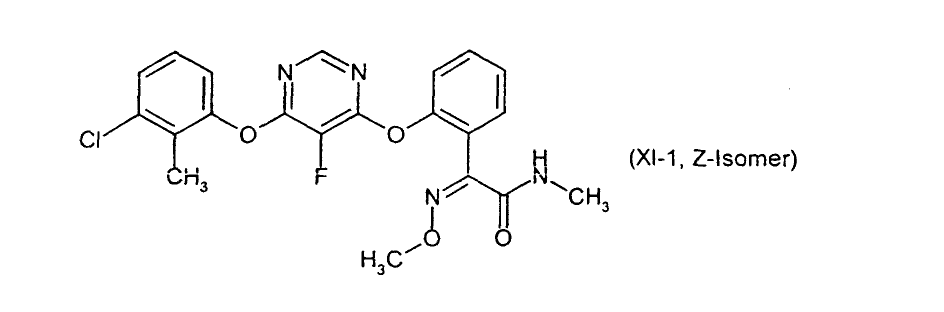

Die Isomerisierung der Verbindungen der Formel (XI) wird bevorzugt im Anschuß an die Verfahrensschritte 10 und 11 durchgeführt.

Als Verdünnungsmittel zur Durchführung der erfindungsgemäßen Verfahren werden beispielhaft und vorzugsweise Alkohole, insbesondere Methanol; Ether, insbesondere Tetrahydrofuran; oder Alkylnitrile, insbesondere Acetonitril verwendet.

Als Verdünnungsmittel zur Durchführung des Verfahrensschritts 1 werden vorzugsweise Ether, insbesondere Tetrahydrofuran; oder Alkohole, insbesondere Ethanol, vorzugsweise Methanol; verwendet.

Als Verdünnungsmittel zur Durchführung des Verfahrensschritts 2 werden vorzugsweise Alkohole, insbesondere Methanol, Pyridin, Wasser oder Mischungen daraus, verwendet.

Als Verdünnungsmittel zur Durchführung des Verfahrensschritts 3 werden vorzugsweise Alkohole, insbesondere Methanol; Dialkylketone, insbesondere Aceton; Dialkylformamide, insbesondere Dimethylformamid, Pyrrolidon, oder Dialkylacetamide, insbesondere Dimethylacetamid, verwendet.

Als Verdünnungsmittel zur Durchführung des Verfahrensschritts 4 werden vorzugsweise Alkylnitrile, insbesondere Acetonitril verwendet.

Als Verdünnungsmittel zur Durchführung des Verfahrensschritts 5 werden vorzugsweise Alkohole, insbesondere Methanol, Pyridin, Wasser oder Mischungen daraus, verwendet.

Als Verdünnungsmittel zur Durchführung des Verfahrensschritts 6 werden vorzugsweise Alkohole, insbesondere Methanol verwendet.

Als Verdünnungsmittel zur Durchführung des Verfahrensschritts 7 werden vorzugsweise Alkohole, insbesondere Methanol verwendet.

Als Verdünnungsmittel zur Durchführung des Verfahrensschritts 8 werden vorzugsweise Alkohole, insbesondere Methanol verwendet.

Als Verdünnungsmittel zur Durchführung des Verfahrensschritts 9 werden vorzugsweise Alkylnitrile, insbesondere Acetonitril, Dialkylketone, insbesondere Aceton; Dialkylformamide, insbesondere Dimethylformamid, Pyrrolidon, oder Dialkyl-acetamide, insbesondere Dimethylacetamid, verwendet.

Als Verdünnungsmittel zur Durchführung des Verfahrensschritts 10 werden vorzugsweise Alkylnitrile, insbesondere Acetonitril, Dialkylketone, insbesondere Aceton; Dialkylformamide, insbesondere Dimethylformamid, Pyrrolidon, oder Dialkylacetamide. insbesondere Dimethylacetamid, verwendet.

Als Verdünnungsmittel zur Durchführung des Verfahrensschritts 11 werden vorzugsweise Alkylnitrile, insbesondere Acetonitril, Dialkylketone, insbesondere Aceton; Dialkylformamide, insbesondere Dimethylformamid, Pyrrolidon, oder Dialkylacetamide. insbesondere Dimethylacetamid. verwendet.

Als Verdünnungsmittel zur Durchführung der Isomerisierung kommen alle inerten organischen Lösungsmittel in Betracht. Hierzu gehören vorzugsweise aromatische Kohlenwasserstoffe, wie beispielsweise Toluol oder Xylol, Ester, wie beispielsweise Essigsäureethylester oder Essigsäure-n-butyl-ester, Ether, wie beispielsweise tert-Butyl-methylether, Dioxan, Tetrahydrofuran oder Dimethoxyethan, Ketone, wie beispielsweise Aceton, Butanon, Cyclohexanon oder Methyl-iso-butylketon, oder Alkohole, wie beispielsweise Methanol, Ethanol, n- oder i-Propanol, n-, i- oder tert-Butanol, oder deren Gemische mit Wasser.

Säuren im Sinne der Erfindung sind höher konzentrierte Säuren, insbesondere Mineralsäuren oder Chlorwasserstoffgas.

Als Mineralsäure ist Salzsäure, insbesondere Chlorwasserstoffgas bevorzugt.

Bei der Isomerisierung werden höher konzentrierte Säuren, insbesondere Mineralsäuren oder Sulfonsäuren, beispielhaft und vorzugsweise Schwefelsäure, Methansulfonsäure, Salzsäure und Chlorwasserstoffgas verwendet.

Als saure Ionenaustauscher werden in den erfindungsgemäßen Verfahren vorzugsweise perfluorierte Ionenaustauscher eingesetzt.

Die erfindungsgemäßen Verfahren werden gegebenenfalls in Gegenwart eines geeigneten Säureakzeptors/Base durchgeführt. Als solche kommen alle üblichen anorganischen oder organischen Basen infrage. Hierzu gehören vorzugsweise Erdalkalimetall- oder Alkalimetallcarbonate, wie beispielsweise Kaliumcarbonat; Erdalkalimetall- oder Alkalimetallhydrogencarbonate, wie beispielsweise Kaliumhydrogencarbonat; primäre Amine, wie Methylamin, tertiäre Amine, wie Trimethylamin, Triethylamin, Tributylamin, N,N-Dimethylanilin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU), besonders bevorzugt Alkaliacetate, insbesondere Natriumacetat.

Im Verfahrensschritt 1 wird vorzugsweise Methylamin eingesetzt.

Im Verfahrensschritt 3 wird vorzugsweise Natriumacetat eingesetzt.

Im Verfahrensschritt 4 wird vorzugsweise Kaliumhydrogencarbonat eingesetzt.

Im Verfahrensschritt 5 wird vorzugsweise Natriumacetat eingesetzt.

Im Verfahrensschritt 9 wird vorzugsweise Kaliumcarbonat eingesetzt.

Im Verfahrensschritt 10 wird vorzugsweise Kaliumcarbonat eingesetzt.

Im Verfahrensschritt 11 wird vorzugsweise Kaliumcarbonat eingesetzt.

Als Alkoxyamine werden im Verfahrensschritt 5 insbesondere Methoxyamin und/oder dessen Hydrochloridsalz eingesetzt.

Als Alkoxyamine werden im Verfahrensschritt 6 insbesondere Methoxyamin und/oder dessen Hydrochloridsalz eingesetzt.

Die Reaktionstemperaturen können bei der Durchführung der erfindungsgemäßen Verfahren in einem grösseren Bereich variiert werden. Im allgemeinen arbeitet man in einem Temperaturbereich von 0°C bis Rückflußtemperatur der jeweiligen Mischung, insbesondere bei Rückflußtemperatur.

Die Umsetzungen nach Verfahrensschritt 1 werden vorzugsweise in einem Temperaturbereich von 0°C bis Raumtemperatur, insbesondere bei 5-15°C durchgeführt.

Die Umsetzungen nach Verfahrensschritt 2 werden vorzugsweise in einem Temperaturbereich von Raumtemperatur bis Rückflußtemperatur der jeweiligen Mischung, insbesondere bei Rückflußtemperatur durchgeführt.

Die Umsetzungen nach Verfahrensschritt 3 werden vorzugsweise in einem Temperaturbereich von Raumtemperatur bis Rückflußtemperatur der jeweiligen Mischung, insbesondere bei Rückflußtemperatur durchgeführt.

Die Umsetzungen nach Verfahrensschritt 4 werden vorzugsweise in einem Temperaturbereich von Raumtemperatur bis Rückflußtemperatur der jeweiligen Mischung, insbesondere bei Raumtemperatur durchgeführt.

Die Umsetzungen nach Verfahrensschritt 5 werden vorzugsweise in einem Temperaturbereich von Raumtemperatur bis Rückflußtemperatur der jeweiligen Mischung, insbesondere bei Rückflußtemperatur durchgeführt.

Die Umsetzungen nach Verfahrensschritt 6 werden vorzugsweise in einem Temperaturbereich von Raumtemperatur bis Rückflußtemperatur der jeweiligen Mischung, insbesondere bei Rückflußtemperatur durchgeführt.

Die Umsetzungen nach Verfahrensschritt 7 werden vorzugsweise in einem Temperaturbereich von Raumtemperatur bis Rückflußtemperatur der jeweiligen Mischung durchgeführt.

Die Umsetzungen nach Verfahrensschritt 8 werden vorzugsweise in einem Temperaturbereich von Raumtemperatur bis Rückflußtemperatur der jeweiligen Mischung durchgeführt.

Die Umsetzungen nach Verfahrensschritt 9 werden vorzugsweise in einem Temperaturbereich von Raumtemperatur bis Rückflußtemperatur der jeweiligen Mischung durchgeführt.

Die Umsetzungen nach Verfahrensschritt 10 werden vorzugsweise in einem Temperaturbereich von Raumtemperatur bis Rückflußtemperatur der jeweiligen Mischung durchgeführt.

Die Umsetzungen nach Verfahrensschritt 11 werden vorzugsweise in einem Temperaturbereich von Raumtemperatur bis Rückflußtemperatur der jeweiligen Mischung durchgeführt.

Die Umsetzungen der erfindungsgemäßen Verfahren erfolgen bei Normaldruck, bei erhöhtem oder bei vermindertem Druck, vorzugsweise bei Normaldruck.

Als Carbonylverbindungen werden vorzugsweise Dialkylketone, insbesondere Aceton, Aldehyde oder Glyoxylsäure verwendet.

Als Alkylierungsmittel werden vorzugsweise Carbonate, insbesondere Dialkylcarbonate, besonders bevorzugt Dimethylcarbonat, Dialkylsulfate, insbesondere Dimethylsulfat, oder besonders bevorzugt Alkylhalogenide, insbesondere Methylchlorid verwendet.

In Verfahrensschritt 9) werden als Pyrimidin-Derivate der Formel (XII) vorzugsweise Trifluorpyrimidin oder Fluordichlorpyrimidine inbesondere 5-Fluor-4,6-Dichlorpyrimidin eingesetzt.

Besonders bevorzugt ist die Durchführung von Verfahren Teil 1A)a) ohne Zwischenisolierung der Verbindungen der Formel (VI), (VII) und (II) (Eintopfverfahren ).

Besonders bevorzugt ist die Durchführung von Verfahren Teil 1A)b) ohne Zwischenisolierung der Verbindungen der Formel (VI) und (II) (Eintopfverfahren).

Besonders bevorzugt ist die Durchführung von Verfahren Teil 1B) ohne Zwischenisolierung der Verbindungen der Formel (II) (Eintopfverfahren).

Besonders bevorzugt ist die Durchführung von Verfahren Teil 1C) ohne Zwischenisolierung der Verbindungen der Formel (VII) und (II) (Eintopfverfahren).

Besonders bevorzugt ist die Durchführung von Verfahren Teil 1D) ohne Zwischenisolierung der Verbindungen der Formel (II) (Eintopfverfahren ).

Besonders bevorzugt ist die Durchführung von Verfahren Teil 1 und Teil 2 ohne Isolierung der Zwischenverbindungen (Eintopfverfahren).

Die zur Durchführung der Verfahrensschritte 2), 6) und 7) verwendeten Ausgangsverbindungen der Formel (IV) sind bekannt und können nach bekannten Verfahren hergestellt werden (vgl. Beilstein, E (II) 17, 462; Mameli, G. 56, 768; Chem. Ber. 35 (1902), 1640-1646; Proc. Indian Acad. Sci. Sect. A (1976) 83A(6), 238-242).

Die zur Durchführung des erfindungsgemäßen Verfahrensschritts 4) als Ausgangsstoffe benötigten Verbindungen der Formel (VII) sind teilweise bekannt (vgl. Giannella; Pigini, FRPSAX, Farmaco Ed.Sci., 28, 1973, 157,159) und werden auf neue Art und Weise nach Verfahrensschritt 7) aus Verbindungen der Formel (IV) oder nach Verfahrensschritt 3) aus Verbindungen der Formel (VI) erhalten.

Die zur Durchführung des bekannten Verfahrensschritts 5) als Ausgangsstoffe benötigten Verbindungen der Formel (VI) sind einerseits bekannt und können nach bekannten Verfahren hergestellt werden, andererseits erhält man sie auf neue Art und Weise nach Verfahrensschritt 2).

Die zur Durchführung des erfindungsgemäßen Verfahrensschritts 3) als Ausgangsstoffe benötigten Verbindungen der Formel (IV) sind bereits bei der Beschreibung des Verfahrensschritts 5) beschrieben worden.

Die im Verfahrensschritt 8) verwendeten Ausgangsverbindungen der Formel (X), in denen R¹, R², R³ und R⁴ für Wasserstoff und R⁵ für Methyl steht, sind in EP-398692 namentlich genannt, die im Verfahrensschritt 8) verwendeten Ausgangsverbindungen der Formel (X), in denen R¹, R², R³ und R⁴ für Wasserstoff und R⁵ für Alkyl steht, sind unter Formel (VIII) auf Seite 8 bzw. Seite 14 und Seite 36 in WO97465 beschrieben worden. Ebenso sind sie unter der Formel (IV) auf Seite 7 und 8 bzw. auf den Seiten 17, 19 und 20 in EP-846691 beschrieben worden.

Die zur Durchführung des Verfahrensschritts 1) verwendeten Ausgangsverbindungen der Formel (II) können nach Verfahren Teil 1Aa), Verfahren Teil 1Ab), Verfahren Teil 1B, Verfahren Teil 1C oder nach Verfahren Teil 1D durch sukzessive Ausführung der Verfahrensschritte oder im Eintopfverfahren hergestellt werden.

Die zur Durchführung der Verfahrensschritte 9), 10) und 11) verwendeten Ausgangsverbindungen sind in WO 98/21189 beschrieben.

Die zur Durchführung der Isomerisierung verwendeten Verbindungen der Formel (XI) werden nach Verfahren Teil 1 und Teil 2 erhalten.

Alle anderen Ausgangsverbindungen sind gängige Handelsprodukte oder können durch einfache Verfahren aus diesen hergestellt werden.

Verfahrensschritt 2 ist neu und ebenfalls Gegenstand der Erfindung.

Nach dem erfindungsgemäßen Verfahrensschritt 3) werden die Verbindungen der Formel (VII) erhalten. Die Verbindungen der Formel (VII) sind neu und erfinderisch und Gegenstand der Erfindung, ausgenommen sind die Verbindungen der Formel (VII-a) 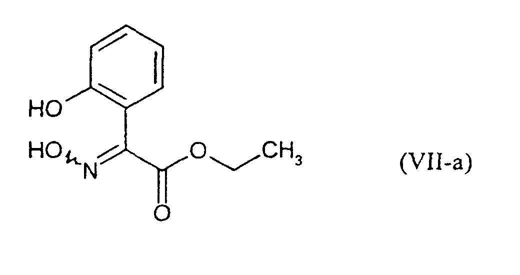 ausgenommen ist.

Nach dem erfindungsgemäßen Verfahrensschritt 7) werden die Verbindungen der Formel (VII) erhalten. Die Verbindungen der Formel (VII) sind neu und erfinderisch und Gegenstand der Erfindung.

Mit Hilfe des Gesamtverfahrens (Verfahren Teil 1 und Verfahren Teil 2) wird die Herstellbarkeit der bekannten Schädlingsbekämpfungsmitteln der Formel (XI) (vgl. WO 98/21189) deutlich verbessert und erleichtert.

Dabei dient das erfindungsgemäße Verfahren Teil 1 zur Herstellung von wichtigen Zwischenprodukten der Formel (1) und liefert diese mit hohen und verbesserten Ausbeuten.

Auch im erfindungsgemäßen Verfahren Teil 2 ist eine im Vergleich zu bekannten Verfahren erhöhte Ausbeute zu beobachten.

Durch die Durchführung der Isomerisierung im Anschluss an Verfahren Teil 2, inbesondere im Anschluss an die Verfahrensschritte 10 und 11 wird der Anteil des E-Isomeren in der Isomerenmischung gesteigert.

Die nachfolgenden Beispiele dienen zur Erläuterung der Erfindung. Die Erfindung ist jedoch nicht auf die Beispiele limitiert.

### Beispiele

### I. Verfahren Teil 1)

### Verfahren Teil 1 A a) (Verfahrensschritte 2, 3, 4 und 1)

### Verfahrensschritt 2

### 2-Hydroxyphenylglyoxylsäuremethylester (Beispiel VI-1a)

6,38 g Acetylchlorid ( 0,081 Mol) werden in eine Mischung aus 65 ml Methanol und 65 ml Aceton eingetropft. Man löst darin 5,2 g (0,032 Mol) Benzofuran-2,3-dion-2-oxim und erhitzt anschließend 1 Stunde unter Rückfluß. Man destilliert das Lösungsmittel im Vakuum ab, gießt den Rückstand auf Wasser, extrahiert mit Diethylether, trocknet die organische Phase über Natriumsulfat und destilliert das Lösungsmittel im Vakuum ab. Man erhält 5,2 g Rohprodukt, das man mit einer Mischung aus 7 Teilen n-Hexan und 3 Teilen Aceton auf Kieselgel chromatographiert. Man erhält 3,8 g eines Produktes, das nach nach HPLC 88 % 2-Hydroxyphenylglyoxylsäuremethyl-ester mit log p = 1,87 enthält.

### Verfahrensschritt 3

### 2-(2-Hydroxyphenyl)-2-hydroxyimino-essigsäureinethylester (Beispiel VII-1a)

3,3 g (0,016 Mol) 2-Hydroxyphenylglyoxylsäuremethylester (HPLC: 88 %, log p = 1,87) (Beispiel VI-1a) werden mit 1,3 g (0,019 Mol) Hydroxylamin Hydrochlorid in 50 ml Methanol eine Stunde unter Rückfluß erhitzt. Man gießt das Reaktionsgemisch auf Wasser, säuert mit 2N Salzsäure an, extrahiert mit Diethylether, trocknet die organische Phase über Natriumsulfat und destilliert das Lösungsmittel im Vakuum ab. Man erhält 2,4 g Rohprodukt, das nach HPLC 17,1 % E-2-(2-Hydroxyphenyl)-2-hydroxyimino-essigsäuremethylester mit log p = 0,95 und 68,9 % Z-2-(2-Hydroxyphenyl)-2-hydroxyimino-essigsäuremethylester mit log p = 1,68 enthält.

### Verfahrensschritt 4

### 2-(2-Hydroxyphenyl)-2-methoxyimino-essigsäuremethylester (Beispiel II-1a)

2,4 g (0,0106 Mol) 2-(2-Hydroxyphenyl)-2-hydroxyimino-essigsäuremethylester (Beispiel VII-1a aus Verfahrensschritt 3; HPLC: 17,1 % E, log p = 0,95; 68,9 % Z, log p = 1,68) werden in 60 ml Acetonitril mit 1,08 g (0,011 Mol) Kaliumhydrogencarbonat und 1,55 g (0,0123 Mol) Dimethylsulfat 10 Stunden am Rückfluß erhitzt. Man gießt das Reaktionsgemisch auf Wasser, extrahiert mit Diethylether und destilliert das Lösungsmittel im Vakuum ab. Man erhält 2,1 g Rohprodukt, das man mit einer Mischung aus 7 Teilen n-Hexan und 3 Teilen Aceton auf Kieselgel chromatographiert. Man erhält 1,6 g Produkt das nach HPLC 36,9 % Z-2-(2-Hydroxyphenyl)-2-methoxyimino-essigsäuremethylester mit log p = 2,39 enthält.

### Verfahren Teil 1 A b) (Verfahrensschritte 2,5 und 1)

### Verfahrensschritt 2

### 2-Hydroxyphenylglyoxylsäuremethylester (Beispiel VI-1)

27 g Acetylchlorid ( 0,344 Mol) werden in eine Mischung aus 245 ml Methanol und 245 ml Aceton eingetropft. Man löst darin 20 g (0,123 Mol) Benzofuran-2,3-dion-2-oxim und erhitzt anschließend 1 Stunde unter Rückfluß. Man destilliert das Lösungsmittel im Vakuum ab, gießt den Rückstand auf Wasser, extrahiert mit Diethylether, trocknet die organische Phase über Natriumsulfat und destilliert das Lösungsmittel im Vakuum ab. Man erhält 20,8 g Rohprodukt, das nach HPLC 92,4 % 2-Hydroxyphenylglyoxylsäuremethylester mit log p = 1,87 enthält.

### Verfahrensschritt 5

### 2-(2-Hydroxyphenyl)-2-methoxyimino-essigsäuremethylester (Beispiel 11-1b)

10,4 g (0,0533 Mol) 2-Hydroxyphenylglyoxylsäuremethylester (HPLC: 92,4 %, log p = 1,87) aus Verfahrensschritt 2 werden mit 7,4 g (0,089 Mol) O-Methylhydroxylamin Hydrochlorid in 150 ml Methanol drei Stunden am Rückfluß gekocht. Man gießt das Reaktionsgemisch auf Wasser, extrahiert mit Diethylether, trocknet die organische Phase über Natriumsulfat und destilliert das Lösungsmittel im Vakuum ab. Man erhält 8,2 g Rohprodukt, das nach HPLC 17,2 % E-2-(2-Hydroxyphenyl)-2-methoxyimino-essigsäuremethylester mit log p = 1,48 und 65,4 % Z-2-(2-Hydroxyphenyl)-2-methoxyimino-essigsäuremethylester mit log p = 2,38 enthält.

### Verfahren Teil 1 B) (Verfahrensschritte 6 und 1)

### Verfahrensschritt 6

### 2-(2-Hydroxyphenyl)-2-methoxyimino-essigsäuremethylester (Beispiel II-1c)

8,2 g (0,05 Mol) Benzofuran-2,3-dion-2-oxim wird mit 9 g (0,108 Mol) O-Methylhydroxylamin Hydrochlorid in 50 ml Methanol 3 Stunden unter Rückfluß erhitzt. Man destilliert das Methanol im Vakuum ab, versetzt den Rückstand mit 50 ml Wasser und extrahiert mit Essigsäureethylester. Man trocknet die organische Phase über Natriumsulfat, destilliert das Lösungsmittel im Vakuum ab und erhält 9,88 g Rohprodukt, das mit Kristallen durchsetzt ist. Es enthält nach HPLC 43,7 % E-2-(2-Hydroxyphenyl)-2-methoxyimino-essigsäuremethylester mit log p = 1,48 und 29.4 % Z-2-(2-Hydroxyphenyl)-2-methoxyimino-essigsäuremethylester mit log p = 2,37.
- ¹H-NMR-Spektrum (DMSO-d₆/TMS):: E-Isomer:3,72 ppm (3H, s); 3,92 ppm (3H, s); Z-Isomer: 3,77 ppm (3H, s); 3,92 ppm (3H, s).

### Verfahren Teil 1 C) (Verfahrensschritte 7, 4 und 1)

### Verfahrensschritt 7

### 2-(2-Hydroxyphenyl)-2-hydroxyimino-essigsäuremethylester (Beispiel VII-1d)

4,9 g Acetylchlorid ( 0,062 Mol) werden in 100 ml Methanol eingetropft. Man löst darin 4,0 g (0,0245 Mol) Benzofuran-2,3-dion-2-oxim und erhitzt anschließend 12 Stunden unter Rückfluß. Man destilliert das Lösungsmittel im Vakuum ab, gießt den Rückstand auf Wasser, extrahiert mit Diethylether, trocknet die organische Phase über Natriumsulfat und destilliert das Lösungsmittel im Vakuum ab. Man erhält 3,9 g Rohprodukt, das nach HPLC 21,7 % E-2-(2-Hydroxyphenyl)-2-hydroxyimino-essigsäuremethylester mit log p = 0,95 und 56 % Z-2-(2-Hydroxyphenyl)-2-hydroxyimino-essigsäuremethylester mit log p = 1,68 enthält.
GC/MS silyliert:
E-Isomeres: 22,0 %, Retentionsindex = 1673, M = 339, 324, 308, 280, 250, 220, 206, 176, 147, 131, 89, 73, 59, 45.
Z-Isomeres: 60,5 %, Retentionsindex = 1744, M = 339, 324, 296, 280, 220, 250, 206, 176,147,131,89,73,59,45.

Dieses Rohprodukt wird mit einer Mischung aus 7 Teilen n-Hexan und 3 Teilen Aceton auf Kieselgel chromatographiert. Man erhält 2,3 g eines Produktes, das nach HPLC 86,4 % Z-2-(2-Hydroxyphenyl)-2-hydroxyimino-essigsäuremethylester mit log p = 1,70 sowie 11,8 % E-Benzofuran-2,3-dion-3-oxim mit log p = 1,55 enthält.

### Verfahrensschritt 4

### 2-(2-Hydroxyphenyl)-2-methoxyimino-essigsäuremethylester (Beispiel II -1d)

1,7 g (0,0075 Mol) 2-(2-Hydroxyphenyl)-2-hydroxyimino-essigsäuremethylester (HPLC: 86,4 % Z, log p = 1,70) (Beispiel VII-1d aus Verfahrensschritt 7) werden in 40 ml Acetonitril mit 0,77 g (0,0076 mMol) Kaliumhydrogencarbonat und 1,1 g (0,0087 Mol) Dimethylsulfat 10 Stunden unter Rückfluß erhitzt. Man gießt das Reaktionsgemisch auf Wasser, extrahiert mit Diethylether und destilliert das Lösungsmittel im Vakuum ab. Man erhält 1,2 g Rohprodukt, das nach HPLC 46 % Z-2-(2-Hydroxyphenyl)-2-methoxyimino-essigsäuremethylester mit log p = 2,38 enthält. Dieses Rohprodukt wird mit einer Mischung aus 7 Teilen n-Hexan und 3 Teilen Aceton auf Kieselgel chromatographiert. Man erhält 0,8 g eines Produktes, das nach HPLC 54,4 % Z-2-(2-Hydroxyphenyl)-2-methoxyimino-essigsäuremethylester mit log p = 2,39 enthält.

### Verfahren Teil 1D) (Verfahrensschritte 8 und 1)

### Verfahrensschritt 8

### 2-(2-Hydroxyphenyl)-2-methoxyimino-essigsäuremethylester (Beispiel II-1e)

5 g Acetylchlorid (0,064 Mol) werden in 50 ml Methanol eingetropft. Man löst darin 4,8 g (0,025 Mol) Benzofuran-2,3-dion-3-(O-methyl-oxim)-2-oxim und erhitzt das Reaktionsgemisch anschließend 12 Stunden unter Rückfluß. Nach dem Abkühlen auf Raumtemperatur gießt man auf Wasser und extrahiert mit Diethylether. Man wäscht die organische Phase mit wäßriger Natriumhydrogencarbonat-Lösung, trocknet über Natriumsulfat und destilliert das Lösungsmittel im Vakuum ab. Man erhält 4,33 g Rohprodukt. Es enthält nach HPLC 27,4 % E-2-(2-Hydroxyphenyl)-2-methoxyimino-essigsäure-methylester mit log p = 1,48 und 56 % Z-2-(2-Hydroxyphenyl)-2-methoxyimino-essigsäuremethylester mit log p = 2,37.
- ¹H-NMR-Spektrum (DMSO-d₆/TMS):: E-Isomer: 3,72 ppm (3H, s); 3,92 ppm (3H, s); Z-Isomer: 3,77 ppm (3H, s) und 3,92 ppm (3H, s).

### Verfahren Teil 1D) (Verfahrensschritte 8 und 1, Eintopfsynthese)

### 2-(2-Hydroxyphenyl)-2-methoxyimino-essigsäure-N-methylamid (Beispiel I-1f)

5 g Acetylchlorid (0,064 Mol) werden in 50 ml Methanol eingetropft. Man löst darin 4,8 g (0,025 Mol) Benzofuran-2,3-dion-3-(O-methyl-oxim)-2-oxim und erhitzt das Reaktionsgemisch anschließend 12 Stunden unter Rückfluß. Anschließend kühlt man auf 0°C, leitet bis zur Sättigung Methylamin ein und läßt das Reaktionsgemisch 12 Stunden bei 10 °C stehen. Man destilliert das Lösungsmittel im Vakuum ab, versetzt den Rückstand mit einer Mischung aus 15 ml 2N Salzsäure und 15 ml gesättigter Kochsalzlösung und extrahiert dreimal mit je 25 ml Essigsäureethylester. Man trocknet die organische Phase über Natriumsulfat, destilliert das Lösungsmittel im Vakuum ab und erhält 6,2 g Rohprodukt. Es enthält nach HPLC 37,9 % E-2-(2-Hydroxyphenyl)-2-methoxyimino-essigsäure-N-methylamid mit log p = 0,98 und 44 % Z-2-(2-Hydroxyphenyl)-2-methoxyiminoessigsäure-N-methylamid mit log p = 1,29.
- ¹H-NMR-Spektrum (DMSO-d₆/TMS):: E-Isomer: 2,67/2,68 ppm (3H, d); 3,86 ppm (3H, s)
Z-Isomer: 2,73/2,75 ppm (3H, d); 3,93 ppm (3H, s).

### Verfahren Teil 1 D) (Verfahrensschritte 8 und 1; Zusatz von Aceton als Carbonylverbindung im Verfahrensschritt 8)

### Verfahrensschritt 8

### 2-(2-Hydroxyphenyl)-2-methoxyimino-essigsäuremethylester (Beispiel II-1g)

10,5 g Acetylchlorid (0,133 Mol) werden in ein Gemisch aus 100 ml Methanol und 100 ml Aceton eingetropft. Man löst darin 10 g (0,052 Mol) Benzofuran-2,3-dion-3-(O-methyl-oxim)-2-oxim und rührt das Reaktionsgemisch anschließend 12 Stunden bei Raumtemperatur. Man gießt die Mischung auf Wasser und extrahiert mit Essigsäureethylester. Man wäscht die organische Phase mit wäßriger Natriumhydrogencarbonat-Lösung, trocknet sie über Natriumsulfat und destilliert das Lösungsmittel im Vakuum ab. Man erhält 9 g Rohprodukt.
Es enthält nach HPLC 17,7 % E-2-(2-Hydroxyphenyl)-2-methoxyimino-essigsäuremethylester mit log p = 1,48 und 61,8 % Z-2-(2-Hydroxyphenyl)-2-methoxyiminoessigsäuremethylester mit log p = 2,38.
- 1H-NMR-Spektrum (DMSO-d6/TMS):: E-Isomer: 3,72 ppm (3H, s); 3,92 ppm (3H, s)
Z-Isomer: 3,77 ppm (3H, s); 3,91 ppm (3H, s).

### Verfahrensschritt 1)

### a) 2-(2-Hydroxyphenyl)-2-methoxyimino-essigsäure-N-methylamid (Beispiel I-1a)

1,2 g (0,0021 Mol) 2-(2-Hydroxyphenyl)-2-methoxyimino-essigsäuremethylester (insbesondere Beispiel II-1a aus Verfahrensschritt 4; HPLC: 36,9 % Z-2-(2-Hydroxyphenyl)-2-methoxyimino-essigsäuremethylester mit log p = 2,39) werden in 14 ml Tetrahydrofuran gelöst und auf 10°C abgekühlt. Man leitet unter Kühlen Methylamin ein. Nach ca. 30 Minuten gibt man 5 ml Methanol zu, sättigt die Lösung mit Methylamin und läßt das Gemisch über Nacht bei 10°C stehen. Man destilliert die Lösungsmittel im Vakuum ab, versetzt den Rückstand mit einer Mischung aus 10 ml 2N Salzsäure und 10 ml gesättigter Kochsalzlösung und extrahiert dreimal mit je 20 ml Essigsäureethylester. Man trocknet die organische Phase über Natriumsulfat, destilliert das Lösungsmittel im Vakuum ab und erhält 1,3 g Rohprodukt. Dieses chromatographiert man mit einer Mischung aus 7 Teilen n-Hexan und 3 Teilen Aceton auf Kieselgel.
Man erhält 0,7 g Produkt, das nach HPLC 37,9 % Z-2-(2-Hydroxyphenyl)-2-methoxyiminoessigsäure-N-methylamid mit log p = 1,32 enthält.

### b) 2-(2-Hydroxyphenyl)-2-methoxyimino-essigsäure-N-methylamid (Beispiel I-1b)

8 g (0,0316 Mol) Rohprodukt (E/Z-2-(2-Hydroxyphenyl)-2-methoxyimino-essigsäuremethylester, insbesondere Beispiel II-1b aus Verfahrensschritt 5; HPLC: 17,2 % E-2-(2-Hydroxyphenyl)-2-methoxyimino-essigsäuremethylester mit log p = 1,48 und 65,4 % Z-2-(2-Hydroxyphenyl)-2-methoxyimino-essigsäuremethylester mit log p = 2,38) werden in 100 ml Tetrahydrofuran gelöst und auf 10 °C abgekühlt. Man leitet unter Kühlen Methylamin ein. Nach ca. 30 Minuten gibt man 30 ml Methanol zu, sättigt die Lösung mit Methylamin und läßt das Gemisch über Nacht bei 10°C stehen. Man destilliert die Lösungsmittel im Vakuum ab, versetzt den Rückstand mit einer Mischung aus 15 ml 2N Salzsäure und 15 ml gesättigter Kochsalzlösung und extrahiert dreimal mit je 25 ml Essigsäureethylester. Man trocknet die organische Phase über Natriumsulfat, destilliert das Lösungsmittel im Vakuum ab und erhält 8,1 g Rohprodukt.
Es enthält nach HPLC 40,3 % E-2-(2-Hydroxyphenyl)-2-methoxyimino-essigsäure-N-methylamid mit log p = 0,98 und 53,1 % Z-2-(2-Hydroxyphenyl)-2-methoxyiminoessigsäure-N-methylamid mit log p = 1,29.

### c) 2-(2-Hydroxyphenyl)-2-methoxyimino-essigsäure-N-methylamid (Beispiel I-1c)

E/Z-2-(2-Hydroxyphenyl)-2-methoxyimino-essigsäuremethylester, insbesondere das nach Verfahrensschritt 6 erhaltene Rohgemisch wird analog dem in EP-398692 beschriebenen Vorgehen (vgl. Beispiel 30, Seite 49, Herstellung des E-Isomeren und des Z-Isomeren von N-Methyl-(2-(2-hydroxy)phenyl)-2-methoxyiminoacetamid aus dem in situ vorliegenden E,Z-2-(2-Hydroxyphenyl)-2-methoxyimino-essigsäuremethylester-Gemisch) wie folgt weiter umgesetzt:

9,68 g Rohprodukt (0,0339 Mol E/Z-2-(2-Hydroxyphenyl)-2-methoxyimino-essigsäuremethylester. insbesondere Verbindung II-1c aus Verfahrensschritt 6, HPLC: 43,7 % E-Isomeres mit log p = 1,48; 29,4 % Z-Isomers mit log p = 2,37) wird in 100 ml Tetrahydrofuran gelöst und auf 10 °C abgekühlt. Man leitet unter Kühlen Methylamin ein. Nach ca. 30 Minuten gibt man 50 ml Methanol zu, sättigt die Lösung mit Methylamin und läßt das Gemisch über Nacht bei 10°C stehen. Man destilliert die Lösungsmittel im Vakuum ab, versetzt den Rückstand mit einer Mischung aus 30 ml 2N Salzsäure und 30 ml gesättigter Kochsalzlösung und extrahiert dreimal mit je 50 ml Essigsäureethylester. Man trocknet die organische Phase über Natriumsulfat, destilliert das Lösungsmittel im Vakuum ab und erhält 8,5 g Rohprodukt.
Es enthält nach HPLC 52,6 % E-2-(2-Hydroxyphenyl)-2-methoxyimino-essigsäure-N-methylamid mit log p = 0,98 und 31,9 % Z-2-(2-Hydroxyphenyl)-2-methoxyiminoessigsäure-N-methylamid mit log p = 1,29.
- ¹H-NMR-Spektrum (DMSO-d₆/TMS):: E-Isomer: 2,67/2,68 ppm (3H, d); 3,86 ppm (3H, s)
Z-Isomer: 2,73/2,75 ppm (3H, d); 3,93 ppm (3H, s).

### d) 2-(2-Hydroxyphenyl)-2-methoxyimino-essigsäure-N-methylamid (Beispiel I-1d)

0,5 g (0,0013 Mol) 2-(2-Hydroxyphenyl)-2-methoxyimino-essigsäuremethylester (insbesondere Beispiel II-1d aus Verfahrensschritt 4; HPLC: 54,4 % Z-2-(2-Hydroxyphenyl)-2-methoxyimino-essigsäuremethylester mit log p = 2,39) werden in 6 ml Tetrahydrofuran gelöst und auf 10°C abgekühlt. Man leitet unter Kühlen Methylamin ein. Nach ca. 30 Minuten gibt man 2 ml Methanol zu, sättigt die Lösung mit Methylamin und läßt das Gemisch über Nacht bei 10°C stehen. Man destilliert die Lösungsmittel im Vakuum ab, versetzt den Rückstand mit einer Mischung aus 5 ml 2N Salzsäure und 5 ml gesättigter Kochsalzlösung und extrahiert dreimal mit je 10 ml Essigsäureethylester. Man trocknet die organische Phase über Natriumsulfat, destilliert das Lösungsmittel im Vakuum ab und erhält 0,6 g Rohprodukt.
Dieses Rohprodukt wird mit einer Mischung aus 7 Teilen n-Hexan und 3 Teilen Aceton auf Kieselgel chromatographiert. Man erhält 0,4 g Produkt, das nach HPLC 99,2 % Z-2-(2-Hydroxyphenyl)-2-methoxyiminoessigsäure-N-methylamid mit log p = 1,29 enthält.

### e) 2-(2-Hydroxyphenyl)-2-methoxyimino-essigsäure-N-methylamid (Beispiel I-1e)

4,33 g (0,0173 Mol) Rohprodukt (E/Z-2-(2-Hydroxyphenyl)-2-methoxyimino-essigsäuremethylester, insbesondere Beispiel II-1e aus Verfahrensschritt 8; HPLC: 27,4 % E-2-(2-Hydroxyphenyl)-2-methoxyimino-essigsäuremethylester mit log p = 1,48 und 56 % Z-2-(2-Hydroxyphenyl)-2-methoxyimino-essigsäuremethylester mit log p = 2,38) werden in 50 ml Tetrahydrofuran gelöst und auf 10°C gekühlt. Man leitet unter Kühlen Methylamin ein. Nach ca. 30 Minuten gibt man 15 ml Methanol zu, sättigt die Lösung mit Methylamin und läßt das Gemisch über Nacht bei 10°C stehen. Man destilliert die Lösungsmittel im Vakuum ab, versetzt den Rückstand mit einer Mischung aus 15 ml 2N Salzsäure und 15 ml gesättigter Kochsalzlösung und extrahiert dreimal mit je 25 ml Essigsäureethylester. Man trocknet die organische Phase über Natriumsulfat, destilliert das Lösungsmittel im Vakuum ab und erhält 4,3 g Rohprodukt.
Es enthält nach HPLC 38,8 % E-2-(2-Hydroxyphenyl)-2-methoxyimino-essigsäure-N-methylamid mit log p = 0,98 und 51 % Z-2-(2-Hydroxyphenyl)-2-methoxyiminoessigsäure-N-methylamid mit log p = 1,29.
- ¹H-NMR-Spektrum (DMSO-d₆/TMS):: E-Isomer: 2,66/2,68 ppm (3H, d); 3,86 ppm (3H, s)
Z-Isomer: 2,73/2,75 ppm (3H, d); 3,93 ppm (3H, s).

### f) 2-(2-Hydroxyphenyl)-2-methoxyimino-essigsäure-N-methylamid (Beispiel 1-1g)

9 g (0,0342 Mol) Rohprodukt (E/Z-2-(2-Hydroxyphenyl)-2-methoxyimino-essigsäuremethylester, insbesondere Beispiel II-1g aus Verfahrensschritt 8; HPLC: 17,7 % E-2-(2-Hydroxyphenyl)-2-methoxyimino-essigsäuremethylester mit log p = 1,48 und 61,8 % Z-2-(2-Hydroxyphenyl)-2-methoxyimino-essigsäuremethylester mit log p = 2,38) werden in 100 ml Tetrahydrofuran gelöst und auf 10°C abgekühlt. Man leitet unter Kühlen Methylamin ein. Nach ca. 30 Minuten gibt man 20 ml Methanol zu, sättigt die Lösung mit Methylamin und läßt das Gemisch über Nacht bei 10°C stehen. Man destilliert die Lösungsmittel im Vakuum ab, versetzt den Rückstand mit einer Mischung aus 30 ml 2N Salzsäure und 30 ml gesättigter Kochsalzlösung und extrahiert dreimal mit je 75 ml Essigsäureethylester. Man trocknet die organische Phase über Natriumsulfat, destilliert das Lösungsmittel im Vakuum ab und erhält 6 g Rohprodukt.
Es enthält nach HPLC 43,7 % E-2-(2-Hydroxyphenyl)-2-methoxyimino-essigsäure-N-methylamid mit log p = 0,98 und 53,5 % Z-2-(2-Hydroxyphenyl)-2-methoxyiminoessigsäure-N-methylamid mit log p = 1,29.

### II. Verfahren Teil 2

### Verfahren Teil 2 E) (Verfahren Teil 1 und Verfahrensschritte 9 und 10)

### unter Verwendung der nach Verfahren Teil 1 D) unter Zusatz von Aceton als Carbonylverbindung im Verfahrensschritt 8 erhaltenen Verbindung (I-1g)

### Verfahrensschritt 9

### 2-(2-(S,6-Difluor-pyrimidin-4-yloxy)-phenyl(-2-methoxyimino-N-methylacetamid (Beispiel XIII-1g) (vgl. Beispiel (IV-1) aus WO9821189, Seite 25)

6 g (0,0280 Mol) 2-(2-Hydroxy-phenyl)-2-methoxyimino-N-methyl-acetamid (Beispiel I-1g aus Verfahrensschritt 1; HPLC: 43,7 % E-2-(2-Hydroxyphenyl)-2-methoxyimino-essigsäure-N-methylamid mit log p = 0,98 und 53,5 % Z-2-(2-Hydroxyphenyl)-2-methoxyiminoessigsäure-N-methylamid mit log p = 1,29) werden in 80 ml Acetonitril gelöst. Man kühlt die Lösung auf 0°C, gibt 4,7 g (0,034 Mol) Kaliumcarbonat dazu und rührt die Mischung 30 Minuten weiter. Dann tropft man 3,8 g (0,0283 Mol) 4,5,6-Trifluorpyrimidin dazu und rührt das Gemisch zwei Stunden bei 20°C. Dann destilliert man das Lösungsmittel im Vakuum ab, versetzt den Rückstand mit Wasser, extrahiert mit Essigsäureethylester; trocknet die organische Phase über Natriumsulfat und destilliert das Lösungsmittel im Vakuum ab. Man erhält 5,7 g (61,4 % der Theorie) rohes 2-[2-(5,6-Difluor-pyrimidin-4-yloxy)-phenyl]2-methoxyimino-N-methylacetamid. Es enthält nach HPLC 45,5 % E-2-[2-(5,6-Difluor-pyrimidin-4-yloxy)-phenyl]-2-methoxyimino-N-methylacetamid mit log p = 1,95 und 46,6 % Z-2-[2-(5,6-Difluor-pyrimidin-4-yloxy)-phenyl]-2-methoxyimino-N-methylacetamid mit log p = 1,87.
- 1H-NMR-Spektrum (CDCl₃/TMS):: E-Isomer: 3,81 ppm (3H, s); 6,67 ppm (1H, b)
Z-Isomer: 3,89 ppm (3H, s); 6,40 ppm (1H,b).

### Verfahrensschritt 10

### 2-[2-(5- Fluor- 2'- methyl-3'-chlorphenoxy-pyrimidin-4-yloxy)-phenyl]-2-methoxyimino-N-methyl-acetamid (Beispiel XI-2g) Verbindungen (XI-1, E-Isomer und XI-1, Z-Isomer)

2,5 g (0,0175 Mol) 2-Methyl-3-chlor-phenol werden in 20 ml Acetonitril gelöst. Man gibt 4,5 g (0,0326 Mol) Kaliumcarbonat dazu und rührt die Mischung 30 Minuten bei Raumtemperatur. Dann kühlt man auf 0°C ab und tropft 5,7 g (0,0163 Mol) 2-[2-(5,6-Difluor-pyrimidin-4-yloy)-phenyl]-2-methoxyimino-N-methylacetamid (Verbindung XIII-1g aus Verfahrensschritt 9, HPLC-Gehalt: 45,5 % E-Isomeres, 46,6 % Z-Isomeres) in Acetonitril gelöst dazu. Man rührt die Mischung 12 Stunden, wobei man die Temperatur auf Raumtemperatur kommen läßt. Man filtriert die Feststoffe ab, engt das Lösungsmittel im Vakuum ein, versetzt den Rückstand mit Wasser und extrahiert mit Essigsäureethylester. Man destilliert das Lösungsmittel im Vakuum ab. Man erhält 2,9 g Produkt. Es enthält nach HPLC 57,8 % E-2-[2-(5-Fluor-2'-methyl-3'-chlorphenoxy-pyrimidin-4-yloxy)-phenyl]-2-methoxyimino-N-methyl-acetamid (XI-1, E-Isomer) mit log p = 3,51 und 30 % Z-2-[2-(5-Fluor-2'methyl-3'-chlorphenoxy-pyrimidin-4-yloxy)-phenyl]-2-methoxyimino-N-methylacetamid (XI-1, Z-Isomer) mit log p = 3,44.

### Verfahren Teil 2 F) (Verfahren 1 und Verfahrensschritt 11)

### Verfahrensschritt 11

### 1. 2-[2-(S-Fluor-2'-chlorphenoxy-pyrimidin-4-yloxy)-phenyl]-2-methoxyimino-N-methyl-acetamid (Beispiel XI-1c, insbesondere unter Verwendung der nach Verfahren Teil 1 B) erhaltenen Verbindung I-1c), Verbindungen (XI-2, E-Isomer und XI-2, Z-Isomer)

8,36 g (0,0339 Mol) rohes E-/Z-2-(2-Hydroxyphenyl)-2-methoxyimino-essigsäure-N-methylamid-Gemisch (I-1c) (HPLC-Gehalt: E-Isomeres: 52,6%, log p = 0,98; Z-Isomeres: 31,9 %, log p = 1,23), das nach Verfahren 1 B) erhalten wurde, werden in 50 ml Acetonitril gelöst und nach Zugabe von 6,7 g (0,048 Mol) Kaliumcarbonat eine Stunde bei Raumtemperatur gerührt. Man tropft 8 g (0,033 Mol) 4-(2'-Chlorphenoxy)-5,6-difluorpyrimidin zum Reaktionsgemisch und rührt 16 Stunden bei Raumtemperatur. Dann tropft man weitere 1,8 g (0,0074 Mol) 4-(2'-Chlor-phenoxy)-5,6-difluorpyrimidin zu und erhitzt anschließend 3 Stunden unter Rückfluß. Man filtriert von den Salzen ab, destilliert das Lösungsmittel im Vakuum ab und verteilt den Rückstand zwischen Essigsäureethylester und wäßriger Natriumcarbonat-Lösung. Man trocknet die organische Phase über Natriumsulfat und destilliert das Lösungsmittel im Vakuum ab. Man erhält 15,3 g Rohprodukt. Es enthält nach HPLC 21 % Z-2-[2-(5-Fluor-2'-chlorphenoxy-pyrimidin-4-yloxy)-phenyl]-2-methoxyimino-N-methyl-acetamid (XI-2, Z-lsomer) mit log p = 2,95 und 54,6 % E-2-[2-(5-Fluor-2'-chlorphenoxy-pyrimidin-4-yloxy)-phenyl]-2-methoxyimino-N-methyl-acetamid (XI-2, E-Isomer) mit log p = 3,01.

### 2. 2-[2-(5-Fluor-2'-chlorphenoxy-pyrimidin-4-yloxy)phenyl]-2-methoxyimino-N-methyl-acetamid (Beispiel XI-1e, insbesondere unter Verwendung der nach Verfahren Teil 1 D) erhaltenen Verbindung I-1e) Verbindungen (XI-2, E-Isomer und XI-2, Z-Isomer)

4,3 g (0,0185 Mol) rohes E-/Z-2-(2-Hydroxyphenyl)-2-methoxyimino-essigsäure-N-methylamid-Gemisch (insbesondere Beispiel I-1e; HPLC: 56 % E, log p = 1,48; 27,4 % Z, log p = 2,38) aus Verfahren 1 D) wird in 100 ml Acetonitril gelöst und nach Zugabe von 3,1 g (0,022 Mol) Kaliumcarbonat 30 Minuten bei Raumtemperatur gerührt. Man tropft 5 g (0,021 Mol) 4-(2'-Chlor-phenoxy)-5,6-difluorpyrimidin zum Reaktionsgemisch und rührt 12 Stunden bei Raumtemperatur. Anschließend kocht man 3 Stunden am Rückfluß. Man gießt auf Wasser, extrahiert mit 3 mal 100 ml Diethylether, trocknet die organische Phase und destilliert das Lösungsmittel im Vakuum ab. Man erhält 7,8 g Rohprodukt. Es enthält nach HPLC 43,9 % Z-2-[2-(5-Fluor-2'-chlorphenoxy-pyrimidin-4-yloxy)-phenyl)-2-methoxyimino-N-methylacetamid (XI-2, Z-Isomer) mit log p = 2,95 und 38,2 % E-2-[2-(5-Fluor-2'chlorphenoxy-pyrimidin-4-yloxy)-phenyl]-2-methoxyimino-N-methyl-acetamid (XI-2, E-Isomer) mit log p = 3,01.

7,8 g rohes 2-[2-(5-Fluor-2'-chlorphenoxy-pyrimidin-4-yloxy)-phenyl]-2-methoxyimino-N-methyl-acetamid (XI-2, Z- und E-Isomer) werden in 50 ml Diethylether gelöst. Man sättigt die Lösung unter Kühlen mit Chlorwasserstoff und läßt das Reaktionsgemisch 2 Tage bei Raumtemperatur stehen. Man destilliert das Lösungsmittel im Vakuum ab, nimmt den Rückstand in Essigsäureethylester auf und wäscht die Lösung mit Wasser. Man trocknet die organische Phase über Natriumsulfat und destilliert das Lösungsmittel im Vakuum ab. Man verrührt den Rückstand mit Diethylether, filtriert ab und erhält 5 g Kristalle, die nach HPLC 97,28 % E-2-[2-(5-Fluor-2'-chlorphenoxy-pyrimidin-4-yloxy)-phenyl]-2-methoxyimino-N-methyl-acetamid (XI-2, E-Isomer) mit log p = 3,01 und 2,94 % Z-2-[2-(5-Fluor-2'chlorphenoxy-pyrimidin-4-yloxy)-phenyl]-2-methoxyimino-N-methyl-acetamid (XI-2, Z-Isomer mit log p = 2,95 enthalten.

### 3. 2-[2-(5-Fluor-2'-methyl-3'-chlorphenoxy-pyrimidin-4-yloxy)-phenyl]-2-methoxyimino-N-methyl-acetamid (Beispiel XI-2f, unter Verwendung der nach Verfahren Teil 1 D) (Eintopfsynthese) erhaltenen Verbindung 1-1f), Verbindungen (XI-1, E-Isomer und XI-1, Z-Isomer)

6 g (0,0236 Mol) rohes E-/Z-2-(2-Hydroxyphenyl)-2-methoxyimino-essigsäure-N-methylamid-Gemisch (insbesondere Beispiel I-1f; HPLC: 37,9 % E, log p = 0,98; 44 % Z, log p = 1,29) wird in 100 ml Acetonitril gelöst und nach Zugabe von 3,5 g (0,025 Mol) Kaliumcarbonat 30 Minuten bei Raumtemperatur gerührt. Man tropft 6 g (0,0234 Mol) 4-(2'-Methyl-3'-chlor-phenoxy)-5.6-difluor-pyrimidin zum Reaktionsgemisch und rührt 12 Stunden bei Raumtemperatur. Anschließend erhitzt man 3 Stunden unter Rückfluß. Man gießt auf Wasser, extrahiert 3 mal mit 100 ml Essigsäureethylester, trocknet die organische Phase über Natriumsulfat. Man sättigt die organische Phase mit Chlorwasserstoff und läßt die Lösung 24 Stunden bei Raumtemperatur stehen. Anschließend destilliert man das Lösungsmittel im Vakuum ab, nimmt den Rückstand in Essigsäureethylester auf und wäscht die organische Phase mit Wasser. Man trocknet sie über Natriumsulfat. destilliert das Lösungsmittel im Vakuum ab und erhält 8,4 g Produkt.
Es enthält nach HPLC 9,4 % Z-2-[2-(5-Fluor-2'-methyl-3'-chlorphenoxy-pyrimidin-4-yloxy)-phenyl]-2-methoxyimino-N-methyl-acetamid (XI-1, Z-Isomer) mit log p = 3,43 und 63,8 % E-2-[2-(5-Fluor-2'-methyl-3'-chlorphenoxy-pyrimidin-4-yloxy)-phenyl]-2-methoxyimino-N-methyl-acetamid (XI-1,E-Isomer) mit log p = 3,50. Man verrührt dieses Produkt mit Diethylether und filtriert ab. Man erhält 4 g Kristalle, die nach HPLC 90,5 % E-2-[2-(5-Fluor-2'-methyl-3'-chlorphenoxypyrimidin-4-yloxy)-phenyl]-2-methoxvimino-N-methyl-acetainid (XI-1, E-Isomer) mit log p = 3,50 und 4,6% Z-2-[2-(5-Fluor-2'-methyl-3'-chlorphenoxy-pyrimidin-4-yloxy)-phenyl]-2-methoxyimino-N-methyl-acetamid (XI-1, Z-Isomer) mit log p = 3,43 enthalten. Man engt die Mutterlauge im Vakuum ein und erhält 3,9 g eines Gemisches, das 30,2 % E-Produkt (XI-1, E-Isomer) mit log p = 3,50 und 13 % Z-Produkt (XI-1, Z-Isomer) mit log p = 3,43 enthält.

### 4. 2-[2-(5-Fluor-2'-chlorphenoxy-pyrimidin-4-yloxy)-phenyl]-2-methoxyimino-N-methyl-acetamid (Beispiel XI-1f, unter Verwendung der nach Verfahren Teil 1 D) (Eintopfsynthese) erhaltenen Verbindung I-1f), Verbindungen (XI-2, E-Isomer und XI-2, Z-Isomer)

5 g (0,0197 Mol) rohes E-/Z-2-(2-Hydroxyphenyl)-2-methoxyimino-essigsäure-N-methylamid-Gemisch (insbesondere Beispiel I-1f; HPLC: 37,9 % E, log p = 0,98; 44 % Z, log p = 1,29) wird in 100 ml Acetonitril gelöst und nach Zugabe von 3,65 g (0,0264 Mol) Kaliumcarbonat 30 Minuten bei Raumtemperatur gerührt. Man tropft 5,87 g (0,0242 Mol) 4-(2'-Chlor-phenoxy)-5,6-difluorpyrimidin zum Reaktionsgemisch und rührt 12 Stunden bei Raumtemperatur. Anschließend erhitzt man 3 Stunden unter Rückfluß. Man gießt auf Wasser, extrahiert 3 mal mit 100 ml Diethylether, trocknet die organische Phase über Natriumsulfat, sättigt sie mit Chlorwasserstoff und läßt sie 24 Stunden bei Raumtemperatur stehen. Anschließend gießt man auf Wasser, trocknet die organische Phase über Natriumsulfat.
Man verrührt den Rückstand mit Diethylether und filtriert ab. Man erhält 4,9 g Kristalle, die nach HPLC 97,6 % E-2-[2-(5-Fluor-2'-chlorphenoxy-pyrimidin-4-yloxy)-phenyl)-2-methoxyimino-N-methyl-acetamid (XI-2, E-Isomer) mit log p = 3,01 und 0,74 % Z-2-[2-(5-Fluor-2'-chlorphenoxy-pyrimidin-4-yloxy)-phenyl]-2-methoxyimino-N-methyl-acetamid (XI-2, Z-Isomer) mit log p = 2,94 enthalten. Man engt die Mutterlauge im Vakuum ein und erhält 2,6 g eines Gemisches, das 10,9 % E-Produkt (XI-2, E-Isomer) mit log p = 3,01 und 17,2 % Z-Produkt mit log p = 2,94 (XI-2, Z-Isomer) enthält.

### III. Vergleichs Beispiele zur Isomerisierung von Verbindungen der allgemeinen Formel (XI)

### 1. E-2-[2-(5-Fluor-2'-methyl-3'-chlorphenoxy-pyrimidin-4-yloxy)-phenyl]-2-methoxymino-N-methyl-acetamid (XI-1 , E-Isomer)

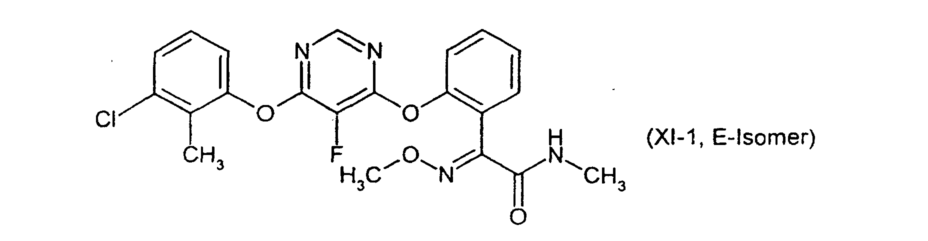

### Isomerisierung von Z-2-[2-(5-Fluor-2'-methyl-3'-chlorphenoxypyrimidin-4-yloxy)-phenyl]-2-methoxyimino-N-methyl-acetamid (XI-1, Z-Isomer) zu E-2-[2-(5-Fluor-2'-methyl-3'-chlorphenoxy-pyrimidin-4-yloxy)-phenyl]-2-methoxyimino-N-methyl-acetamid (XI-1, E-Isomer)

Toluol (10 ml) wird bei Raumtemperatur mit Chlorwasserstoffgas gesättigt und anschließend mit (2Z)-2-(2-{[6-(3-chlor-2-methylphenoxy)-5-fluor-4-pyrimidinyl]-oxy}phenyl)-2-(methoxyimino)-N-methylethanamid (0.5 g. Gehalt (Z+E): 99.3 %, Z/E = 99/1) (XI-1-Z-Isomer) versetzt. Man rührt das Reaktionsgemisch 24 Stunden bei Raumtemperatur, destilliert das Lösungsmittel ab und erhält E-2-[2-(5-Fluor-2'-methyl-3'-chlorphenoxypyrimidin-4-yloxy)-phenyl]-2-methoxyimino-N-methyl-acetamid (XI-1, E-Isomer) (0.5 g. Gehalt (E+Z): 98.3 %, HPLC E/Z = 92/8).

### 2. E-2-[2-(5-Fluor-2'-methyl-3'-chlorphenoxy-pyrimidin-4-yloxy)-phenyl]-2-methoxyimino-N-methyl-acetamid (XI-1, E-Isomer)

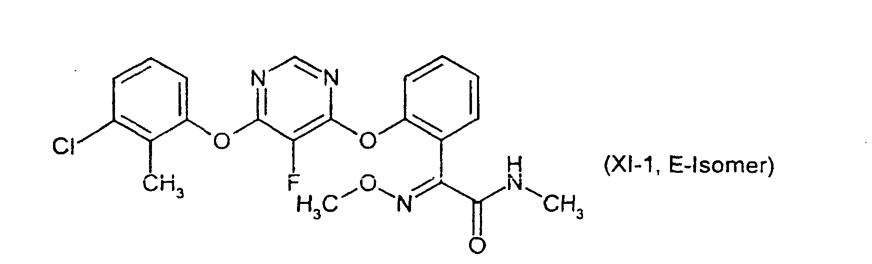

### Isomerisierung von Z-2-[2-(5-Fluor-2'-methyl-3'-chlorphenoxypyrimidin-4-yloxy)-phenyl]-2-methoxyimino-N-methyl-acetamid (XI-1, Z-Isomer) zu E-2-[2-(5-Fluor-2'-methyl-3'-chlorphenoxy-pyrimidin-4-yloxy)-phenyl]-2-methoxyimino-N-methyl-acetamid (XI-1, E-Isomer)

(2Z)-2-(2-{[6-(3-chlor-2-methylphenoxy)-5-fluor-4-pyrimidinyl]oxy}-phenyl)-2-(methoxy-imino)-N-methylethanamid (0.5 g, Gehalt (Z+E): 99.3 %, Z/E = 99/1) (XI-1, Z-Isomer) wird in Essigester (9.5 g) suspendiert und mit konz. Schwefelsäure (0.5 g) versetzt. Man rührt 20 Stunden bei Raumtemperatur, destilliert das Lösungsmittel im Vakuum ab, nimmt den Rückstand mit Methylenchlorid/Wasser auf, trennt die organische Phase ab und trocknet diese über Natriumsulfat. Nach Abdestillieren des Lösungsmittels wird E-2-[2-(5-Fluor-2'-methyl-3'-chlorphenoxypyrimidin-4-yloxy)-phenyl]-2-methoxyimino-N-methyl-acetamid (XI-1, E-Isomer) (0.4 g, Gehalt (E+Z): 98.4 %, HPLC E/Z = 96/4) erhalten.

Die Bestimmung der logP-Werte erfolgte bei allen Beispielen gemäß EEC-Direktive 79/831 Annex V. A8 durch HPLC (Gradientenmethode, Acetonitril/0,1 % wäßrige Phosphorsäure).

## Patentansprüche

1. Verfahren zur Herstellung von Verbindungen der Formel (I), 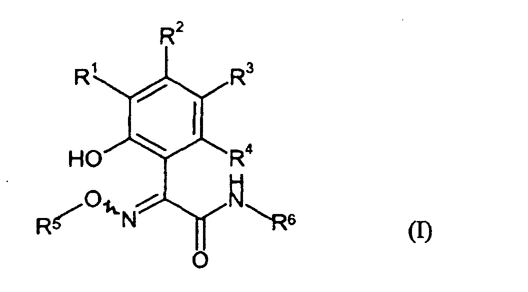 in welcher
R¹, R², R³ und R⁴ gleich oder verschieden sind und unabhängig voneinander jeweils für Wasserstoff, Halogen, Cyano, Nitro, jeweils gegebenenfalls durch Halogen substituiertes Alkyl, Alkoxy, Alkylthio, Alkylsulfinyl oder Alkylsulfonyl stehen,
R⁵ für substituiertes oder unsubstuiertes Alkyl steht,
R⁶ für Wasserstoff, substituiertes oder unsubstituiertes Alkyl steht,
**dadurch gekennzeichnet, dass** man
A) nach Verfahrensschritt 2) Verbindungen der Formel (IV), 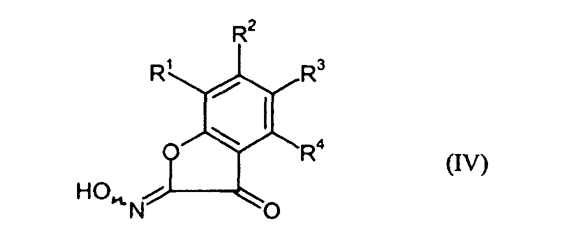 in welcher
R¹, R², R³ und R⁴ die oben angegebenen Bedeutungen haben,
in Gegenwart einer Säure oder eines sauren Ionenaustauschers mit einem Alkohol der Formel (V),
R⁷-OH (V)
in welcher
R⁷ für substituiertes oder unsubstituiertes Alkyl steht,
und mit einer Carbonylverbindung, die das bei der Reaktion abgespaltene Hydroxylammoniumchlorid unter Oximbildung bindet, zu Verbindungen der Formel (VI) umsetzt, 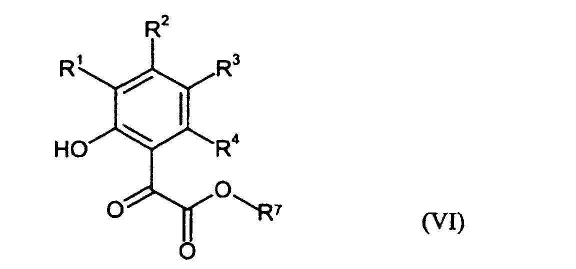 in welcher
R¹, R², R³, R⁴ und R⁷ die oben angegebenen Bedeutungen haben,
und die so erhaltenen Verbindungen der Formel (VI) entweder
a) nach Verfahrensschritt 3) mit einem Hydroxylammoniumsalz gegebenenfalls in Gegenwart eines Verdünnungsmittels und gegebenenfalls in Gegenwart einer Säure oder eines Säureakzeptors zu Verbindungen der Formel (VII), 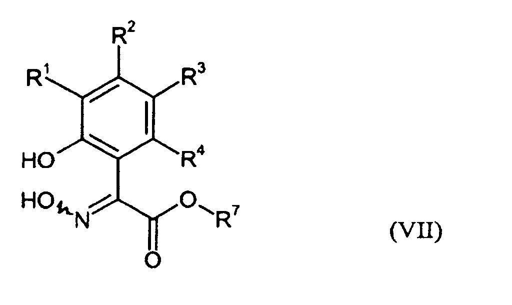 in welcher
R¹, R², R³, R⁴ und R⁷ die oben angegebenen Bedeutungen haben,
umsetzt, und die so erhaltenen Verbindungen der Formel (VII) nach Verfahrensschritt 4) mit einem Alkylierungsmittel der Formel (VIII),
R⁵-X (VIII)
in welcher
R⁵ die oben angegebene Bedeutung hat, und
X für Halogen oder -O-SO₂-O-R⁵ steht, wobei R⁵ die oben angegebene Bedeutung hat,
gegebenenfalls in Gegenwart eines Verdünnungsmittels und gegebenenfalls in Gegenwart einer Base umsetzt,
oder
b) nach Verfahrenschritt 5) mit einem Alkoxyamin der Formel (IX),
R⁵-O-NH₂ (IX)
in welcher
R⁵ die oben angegebene Bedeutung hat,
- oder einem Säureadditionskomplex davon - gegebenenfalls in Gegenwart eines Verdünnungsmittels und gegebenenfalls in Gegenwart einer Säure oder eines Säureakzeptors umsetzt,
oder dadurch, dass man
B) nach Verfahrensschritt 6) Verbindungen der Formel (IV), 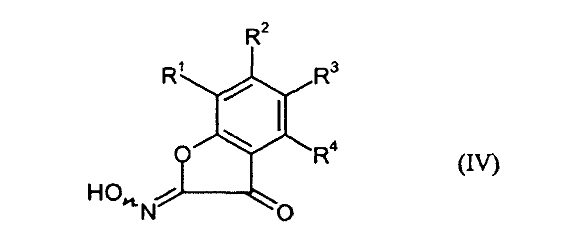 in welcher
R¹, R², R³ und R⁴ die oben angegebenen Bedeutungen haben,
mit einem Alkoxyamin der Formel (IX),
R⁵-O-NH₂ (IX)
in welcher
R⁵ die oben angegebene Bedeutung hat,
- oder einem Säureadditionskomplex davon - gegebenenfalls in Gegenwart eines Verdünnungsmittels und gegebenenfalls in Gegenwart einer Säure umsetzt,
oder dadurch, dass man
C) nach Verfahrensschritt 7) Verbindungen der Formel (IV), 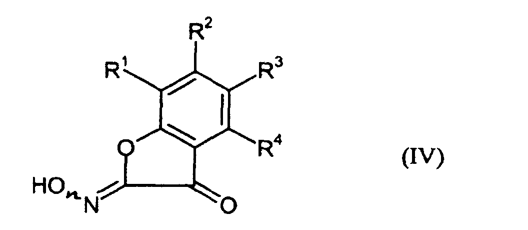 in welcher
R¹, R², R³ und R⁴ die oben angegebenen Bedeutungen haben,
in Gegenwart einer Säure oder eines sauren Ionenaustauschers mit einem Alkohol der Formel (V),
R⁷-OH (V)
in welcher R⁷ die oben angegebene Bedeutung hat,
gegebenenfalls unter Zusatz eines Hydroxylammoniumsalzes umsetzt, und die so erhaltenen Verbindungen der Formel (VII), 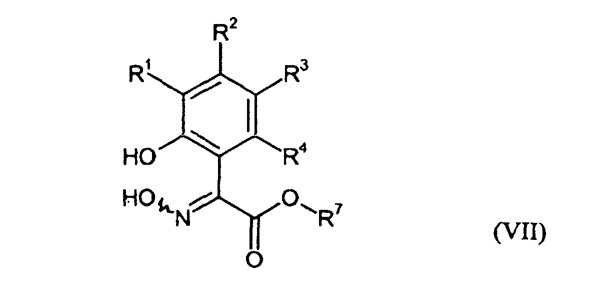 in welcher
R¹, R², R³, R⁴ und R⁷ die oben angegebenen Bedeutungen haben,
nach Verfahrensschritt 4) umsetzt,
oder dadurch, dass man
D) nach Verfahrensschritt 8) Verbindungen der Formel (X), 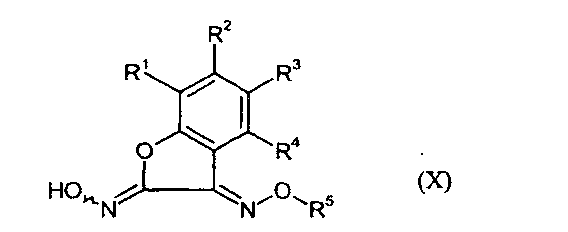 in welcher
R¹, R², R³, R⁴ und R⁵ die oben angegebenen Bedeutungen haben,
in Gegenwart einer Säure oder eines sauren Ionenaustauschers mit einem Alkohol der Formel (V),
R⁷-OH (V)
in welcher R⁷ die oben angegebene Bedeutung hat,
gegebenenfalls in Anwesenheit einer Carbonylverbindung, die das bei der Reaktion abgespaltene Hydroxylammoniumchlorid unter Oximbildung bindet, umsetzt,
und die nach A)-D) erhaltenen Verbindungen der Formel (II), 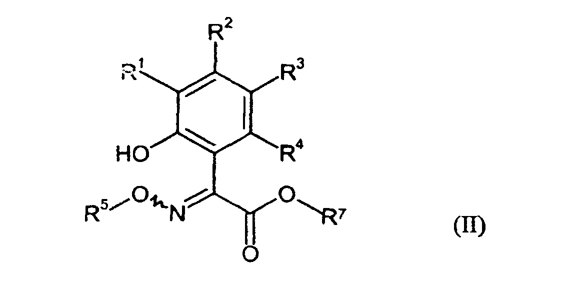 in welcher
R¹, R², R³, R⁴ und R⁵ die oben angegebenen Bedeutungen haben, und
R⁷ für unsubstituiertes oder substituiertes Alkyl steht,
gegebenenfalls ohne Zwischenisolierung der Verbindungen der Formel (II) (Eintopfverfahren),
nach Verfahrensschritt 1) mit einem Alkylamin der Formel (III),
R⁶-NH₂ (III)
in welcher R⁶ die oben angegebene Bedeutung hat, gegebenenfalls in Gegenwart eines Verdünnungsmittels umsetzt.

2. Verfahren gemäß Anspruch 1, **dadurch gekennzeichnet, dass** Verfahren Teil 1Ab) (Verfahrensschritte 2, 5, und 1) als Eintopfverfahren durchgeführt wird.

3. Verfahren gemäß Anspruch 1, **dadurch gekennzeichnet, dass** als Verdünnungsmittel in allen Verfahrensschritten Methanol eingesetzt wird.

4. Verfahren gemäß Anspruch 1, **dadurch gekennzeichnet, dass** als Alkylierungsmittel Methylchlorid verwendet wird.

5. Verfahren zur Herstellung von Verbindungen der Formel (XI), 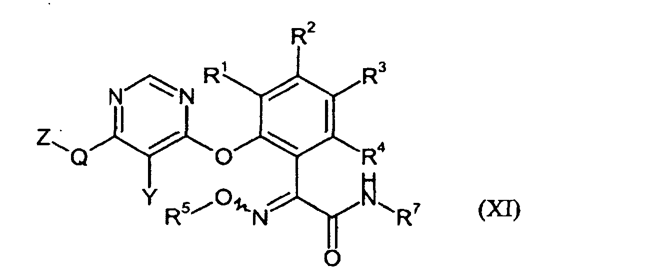 in welcher
Z für unsubstituiertes oder substituiertes Cycloalkyl, Aryl oder Heterocyclyl steht,
Q für Sauerstoff oder Schwefel steht,
Y für Halogen steht,
R¹, R², R³ und R⁴ gleich oder verschieden sind und unabhängig voneinander jeweils für Wasserstoff, Halogen, Cyano, Nitro, jeweils gegebenenfalls durch Halogen substituiertes Alkyl, Alkoxy, Alkylthio, Alkylsulfinyl oder Alkylsulfonyl stehen,
R⁵ für substituiertes oder unsubstuiertes Alkyl steht, und
R⁷ für unsubstituiertes oder substituiertes Alkyl steht,
**dadurch gekennzeichnet, dass** man nach Anspruch 1 hergestellten Verbindungen der Formel (I) entweder
E) nach Verfahrensschritt 9) mit Pyrimidin-Derivaten der Formel (XII), 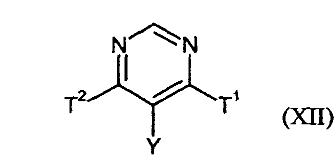 in welcher
T¹ und T² gleich oder verschieden sind und für Halogen stehen, und
Y die oben angegebene Bedeutung hat,
gegebenenfalls in Gegenwart eines Verdünnungsmittels und gegebenenfalls in Gegenwart einer Base umsetzt, und die so erhaltenen Verbindungen der Formel (XIII), 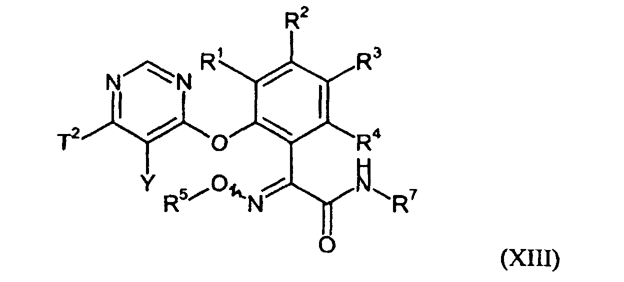 in welcher
T²,Y , R¹, R², R³, R⁴,R⁵ und R⁷ die oben angegebenen Bedeutungen haben,
nach Verfahrensschritt 10) mit einer Ringverbindung der allgemeinen Formel (XIV),
Z-Q-H (XIV)
in welcher
Z und Q die oben angegebenen Bedeutungen haben,
gegebenenfalls in Gegenwart eines Verdünnungsmittels und gegebenenfalls in Gegenwart eines Säureakzeptors und gegebenenfalls in Gegenwart eines Katalysators umsetzt,
oder
F) nach Verfahrensschritt 11) mit Verbindungen der Formel (XV), 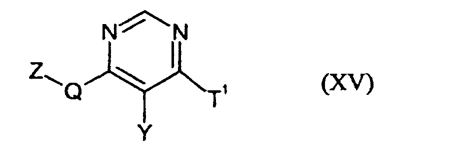
in welcher
Z, Q, T¹ und Y die oben angegebenen Bedeutungen haben,
gegebenenfalls in Gegenwart eines Verdünnungsmittels und gegebenenfalls in Gegenwart einer Base umsetzt.

6. Verfahren gemäß Anspruch 5, **dadurch gekennzeichnet, dass** man die. nach Anspruch 1 hergestellten Verbindungen der Formel I entweder nach Verfahrensschritt 9), 10) oder 11) ohne Isolierung der Zwischenverbindungen umsetzt.

7. Verbindungen, der Formel (VII), 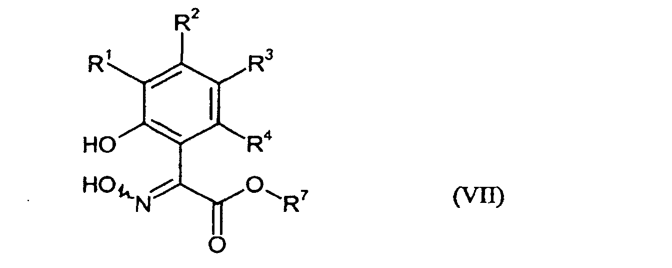 in welcher
R¹, R², R³, R⁴ und R⁷ die in Anspruch 1 angegebenen Bedeutungen haben, wobei
die Verbindung (VII-a) 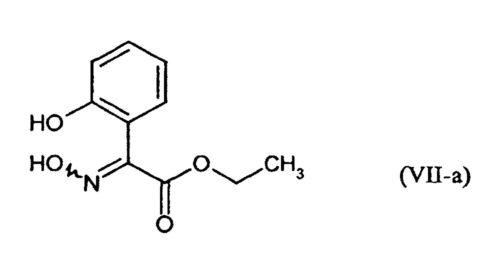 ausgenommen ist.

8. Verwendung von Verbindungen der Formel (VII) wie in Anspruch 7 definiert als Zwischenprodukte zur Herstellung von Verbindungen der Formel (I) wie in Anspruch 1 definiert.

9. Verfahren zur Herstellung von Verbindungen der Formel (VI), 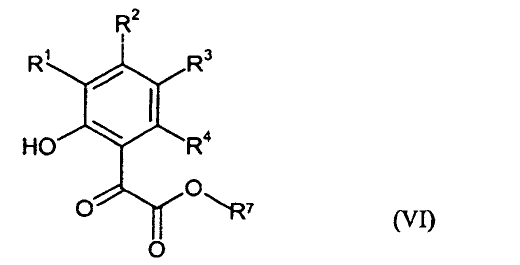 in welcher
R¹, R², R³ und R⁴ gleich oder verschieden sind und unabhängig voneinander jeweils für Wasserstoff, Halogen, Cyano, Nitro, jeweils gegebenenfalls durch Halogen substituiertes Alkyl, Alkoxy, Alkylthio, Alkylsulfinyl oder Alkylsulfonyl stehen, und
R⁷ für unsubstituiertes oder substituiertes Alkyl steht,
**dadurch gekennzeichnet, dass** man Verbindungen der Formel (IV), 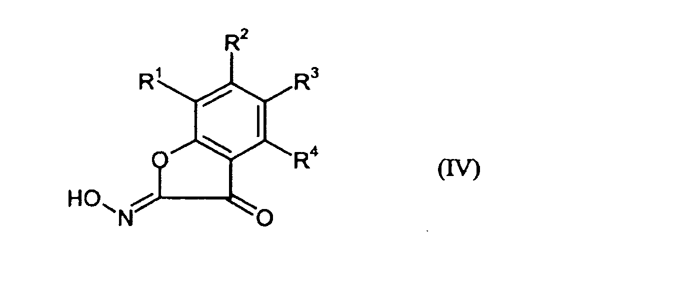 in welcher
R¹, R², R³ und R⁴ die oben angegebenen Bedeutungen haben,
in Gegenwart einer Säure oder eines sauren Ionenaustauschers mit einem Alkohol der Formel (V),
R⁷-OH (V)
in welcher R⁷ die oben angegebene Bedeutung hat, und mit einer Carbonylverbindung, die das bei der Reaktion abgespaltene Hydroxylammoniumchlorid unter Oximbildung bindet, umsetzt.

10. Verbindung der Formel (XI-1, E-Isomer)

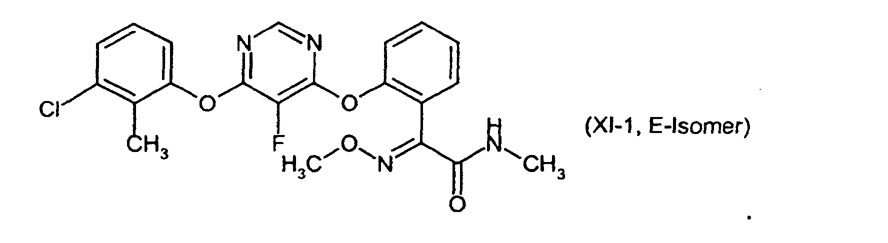

11. Verbindung der Formel (XI-1, Z-Isomer)

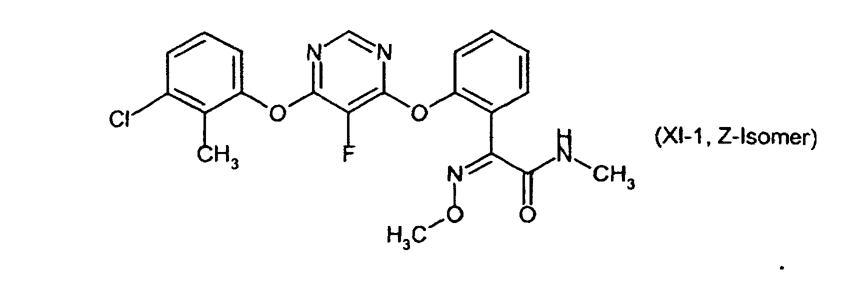

## Claims

1. Process for preparing compounds of the formula (I), 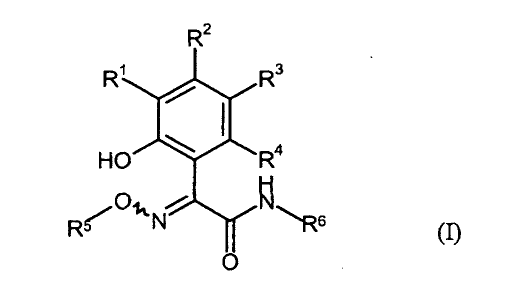 in which
R¹, R², R³ and R⁴ are identical or different and independently of one another each represents hydrogen, halogen, cyano, nitro, in each case optionally halogen-substituted alkyl, alkoxy, alkylthio, alkylsulphinyl or alkylsulphonyl,
R⁵ represents substituted or unsubstituted alkyl,
R⁶ represents hydrogen, substituted or unsubstituted alkyl,
**characterized in that**
A) according to process step 2), compounds of the formula (IV), 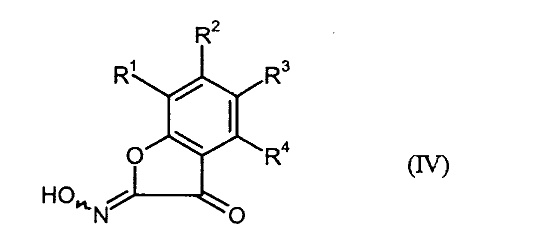 in which
R¹, R², R³ and R⁴ are as defined above,
are reacted, in the presence of an acid or an acidic ion exchanger, with an alcohol of the formula (V),
R⁷-OH (V)
in which
R⁷ represents substituted or unsubstituted alkyl,
and with a carbonyl compound, which binds the hydroxylammonium chloride eliminated in the reaction forming an oxime, to give compounds of the formula (VI), 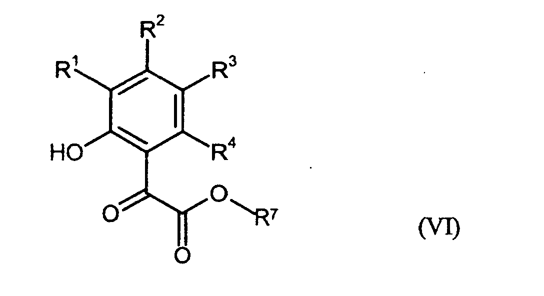 in which
R¹, R², R³, R⁴ and R⁷ are as defined above,
and the resulting compounds of the formula (VI) are either
a) according to process step 3), reacted with a hydroxylammonium salt, if appropriate in the presence of a diluent and if appropriate in the presence of an acid or an acid acceptor, to give compounds of the formula (VIII), 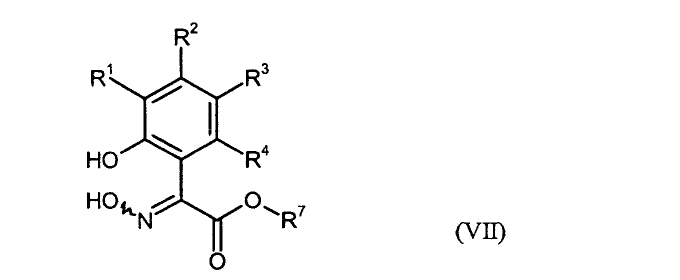 in which
R¹, R², R³, R⁴ and R⁷ are as defined above,
and the resulting compounds of the formula (VII) are, according to process step 4), reacted with an alkylating agent of the formula (VIII),
R⁵-X (VIII)
in which
R⁵ is as defined above and
X represents halogen or -O-SO₂-O-R⁵, where R⁵ is as defined above,
if appropriate in the presence of a diluent and if appropriate in the presence of a base,
or
b) are, according to process step 5), reacted with an alkoxyamine of the formula (IX),
R⁵-O-NH₂ (IX)
in which
R⁵ is as defined above,
- or an acid addition complex thereof -, if appropriate in the presence of a diluent and if appropriate in the presence of an acid or an acid acceptor,
or **in that**
B) according to process step 6), compounds of the formula (IV), 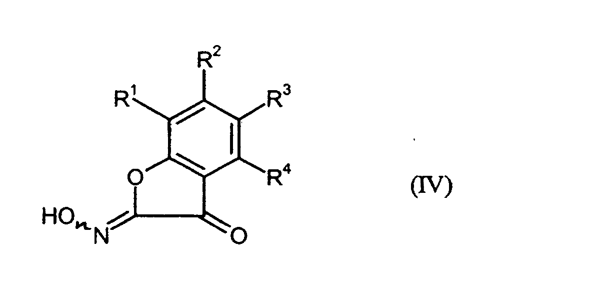 in which
R¹, R², R³ and R⁴ are as defined above,
are reacted with an alkoxyamine of the formula (IX),
R⁵-O-NH₂ (IX)
in which
R⁵ is as defined above,
- or an acid addition complex thereof - if appropriate in the presence of a diluent and if appropriate in the presence of an acid,
or **in that**
C) according to process step 7), compounds of formula (IV), 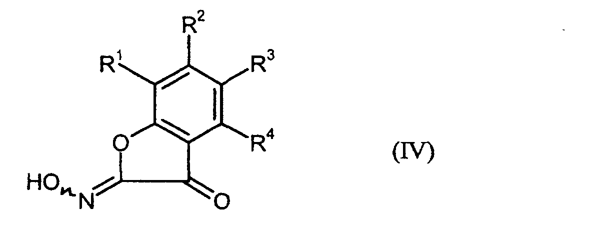 in which
R¹, R², R³ and R⁴ are as defined above,
are reacted, in the presence of an acid or an acidic ion exchanger, with an alcohol of the formula (V),
R⁷-OH (V)
in which R⁷ is as defined above,
if appropriate with addition of a hydroxylammonium salt, and the resulting compounds of the formula (VII), 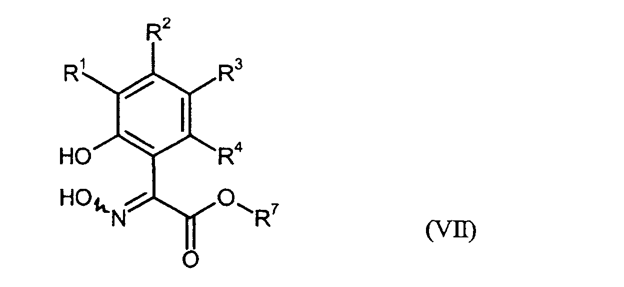 in which
R¹, R², R³, R⁴ and R⁷ are as defined above,
are reacted according to process step 4),
or **in that**
D) according to process step 8), compounds of the formula (X), 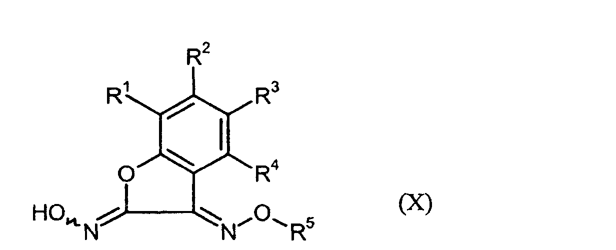 in which
R¹, R², R³, R⁴ and R⁵ are as defined above,
are reacted, in the presence of an acid or an acidic ion exchanger, with an alcohol of the formula (V),
R⁷-OH (V)
in which R⁷ is as defined above,
if appropriate in the presence of a carbonyl compound which binds the hydroxylammonium chloride eliminated in the reaction forming an oxime,
and the compounds of the formula (II) obtained according to A)-D), 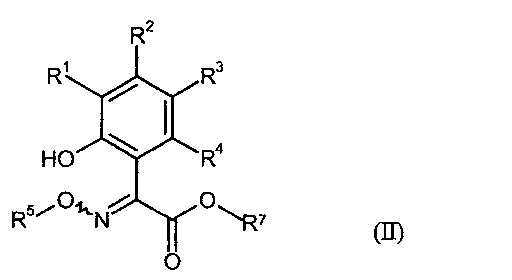 in which
R¹, R², R³, R⁴ and R⁵ are as defined above and
R⁷ represents unsubstituted or substituted alkyl,
are, if appropriate without intermediate isolation of the compounds of the formula (II) (one-pot process),
reacted according to process step 1) with an alkylamine of the formula (III),
R⁶-NH₂ (III)
in which R⁶ is as defined above, if appropriate in the presence of a diluent.

2. Process according to Claim 1, **characterized in that** process part 1Ab) (process steps 2, 5, and 1) are carried out as a one-pot process.

3. Process according to Claim 1, **characterized in that** the diluent used in all process steps is methanol.

4. Process according to Claim 1, **characterized in that** the alkylating agent used is methyl chloride.

5. Process for preparing compounds of the formula (XI), 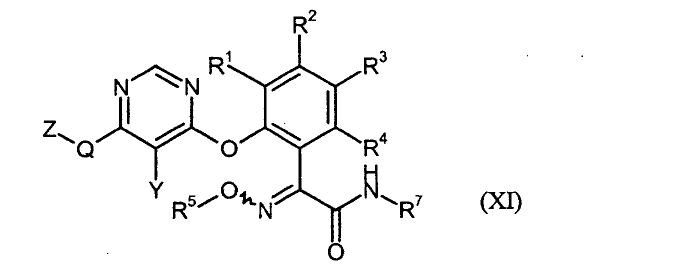 in which
Z represents unsubstituted or substituted cycloalkyl, aryl or heterocyclyl,
Q represents oxygen or sulphur,
Y represents halogen,
R¹, R², R³ and R⁴ are identical or different and independently of one another each represents hydrogen, halogen, cyano, nitro, in each case optionally halogen-substituted alkyl, alkoxy, alkylthio, alkylsulphinyl or alkylsulphonyl,
R⁵ represents substituted or unsubstituted alkyl and
R⁷ represents unsubstituted or substituted alkyl,
**characterized in that** compounds of the formula (I) prepared according to Claim 1 are either
E) according to process step 9) reacted with pyrimidine derivatives of the formula (XH), 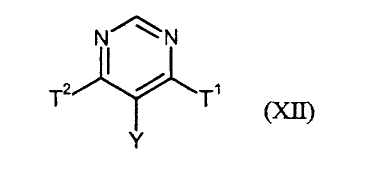 in which
T¹ and T² are identical or different and represent halogen and
Y is as defined above,
if appropriate in the presence of a diluent and if appropriate in the presence of a base, and the resulting compounds of the formula (XIII), 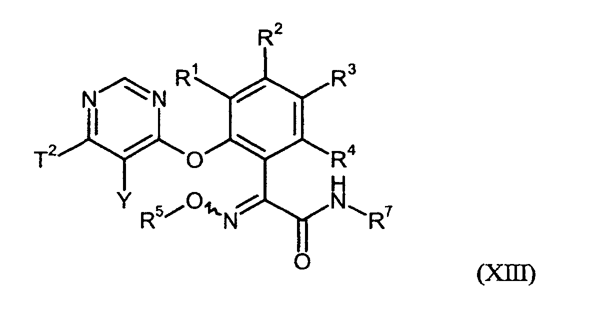 in which
T²,Y , R¹, R², R³, R⁴, R⁵ and R⁷ are as defined above,
are reacted, according to process step 10), with a cyclic compound of the general formula (XIV),
Z-Q-H (XIV)
in which
Z and Q are as defined above,
if appropriate in the presence of a diluent and if appropriate in the presence of an acid acceptor and if appropriate in the presence of a catalyst,
or
F) are reacted according to process step 11) with compounds of the formula (XV), 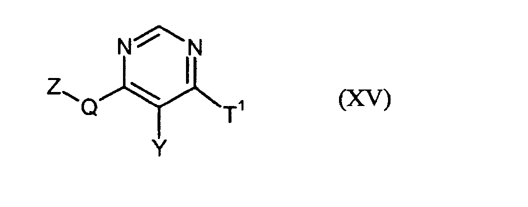 in which
Z, Q, T¹ and Y are as defined above,
if appropriate in the presence of a diluent and if appropriate in the presence of a base.

6. Process according to Claim 5, **characterized in that** the compounds of the formula (I) prepared according to Claim 1 are reacted according to process step 9), 10) or 11), without isolation of the intermediate compounds.

7. Compounds of the formula (VII), 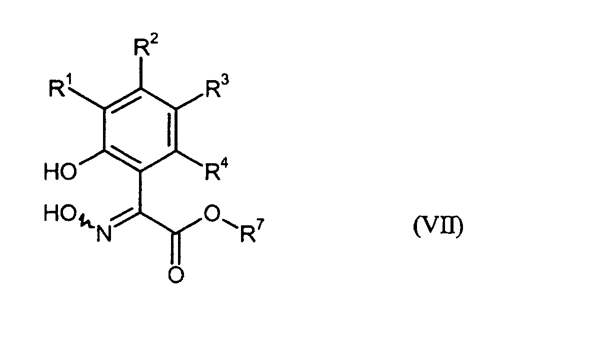 in which
R¹, R², R³, R⁴ and R⁷ are as defined in claim 1,
except for the compound (VII-a)

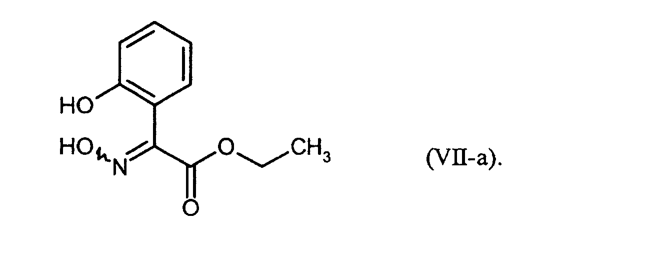

8. Use of compounds of the formula (VII) as defined in Claim 7 as intermediates for preparing compounds of the formula (I) as defined in Claim 1.

9. Process for preparing compounds of the formula (VI), 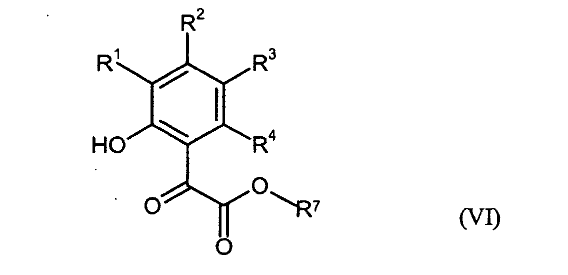 in which
R¹, R², R³ and R⁴ are identical or different and independently of one another each represents hydrogen, halogen, cyano, nitro, in each case optionally halogen-substituted alkyl, alkoxy, alkylthio, alkylsulphinyl or alkylsulphonyl and
R⁷ represents unsubstituted or substituted alkyl,
**characterized in that** compounds of the formula (IV), 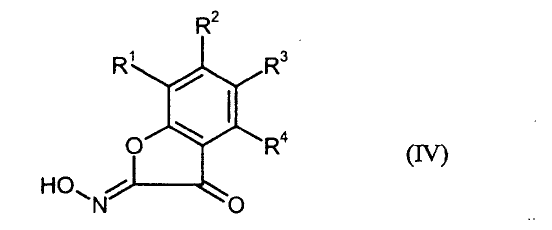 in which
R¹, R², R³ and R⁴ are as defined above,
are reacted, in the presence of an acid or an acidic ion exchanger, with an alcohol of the formula (V),
R⁷-OH (V)
in which R⁷ is as defined above and with a carbonyl compound which binds the hydroxylammonium chloride eliminated in the reaction forming an oxime.

10. Compound of the formula (XI-1, E isomer)

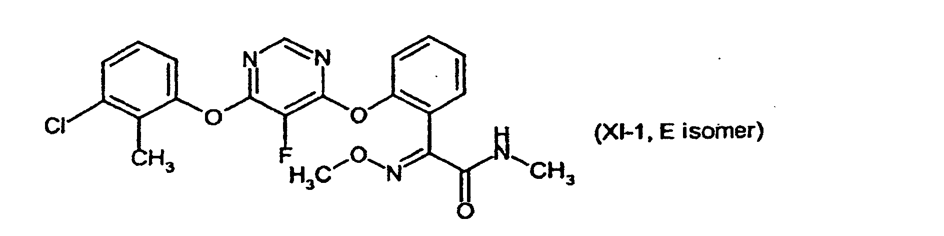

11. Compound of the formula (XI-1, Z isomer)

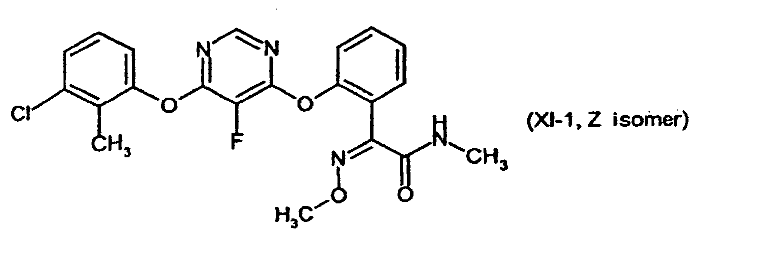

## Revendications

1. Procédé de préparation de composés de la formule (I): 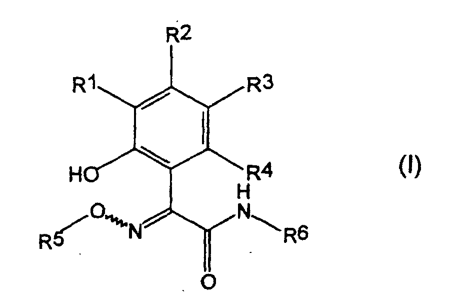 dans laquelle:
R¹, R², R³ et R⁴ sont identiques ou différents et représentent indépendamment l'un de l'autre, chaque fois l'atome d'hydrogène, un atome d'halogène, le radical cyano, nitro, un radical alcoyle, alcoxy, alcoylthio, alcoylsulfinyle ou alcoylsulfonyle chaque fois le cas échéant substitué par des atomes d'halogène,
R⁵ représente un radical alcoyle substitué ou non substitué,
R⁶ représente l'atome d'hydrogène ou un radical alcoyle substitué ou non substitué,
**caractérisé en ce que**
A) selon l'étape de procédé 2), on fait réagir des composés de la formule (IV): 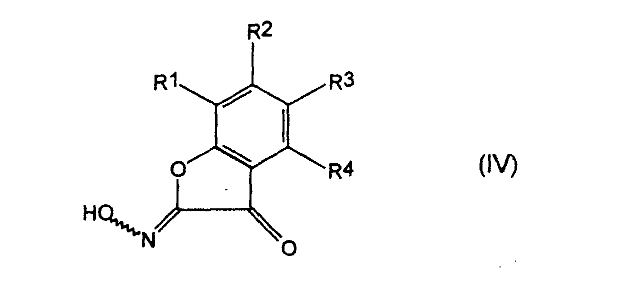 dans laquelle :
R¹, R², R³ et R⁴ ont les significations indiquées ci-dessus,
en présence d'un acide ou d'un échangeur d'ions acide, avec un alcool de la formule (V) :
R⁷ - OH (V)
dans laquelle
R⁷ représente un radical alcoyle substitué ou non substitué,
et avec un composé carbonylé, qui lie le chlorure d'hydroxylammonium clivé lors de la réaction pour former un oxime, en des composés de la formule (VI): 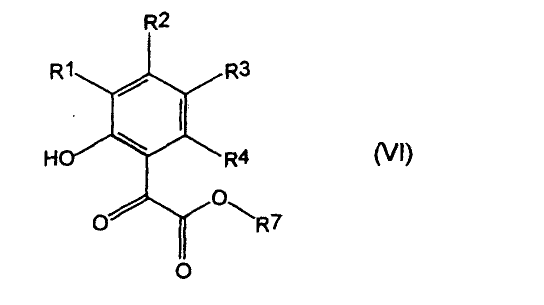 dans laquelle:
R¹, R², R³, R⁴ et R⁷ ont les significations indiquées ci-dessus,
et les composés ainsi obtenus de la formule (VI):
a) selon l'étape de procédé 3), sont mis à réagir avec un sel d'hydroxylammonium, le cas échéant en présence d'un agent de dilution et le cas échéant, en présence d'un acide ou d'un accepteur d'acide, en les composés de la formule (VII) : 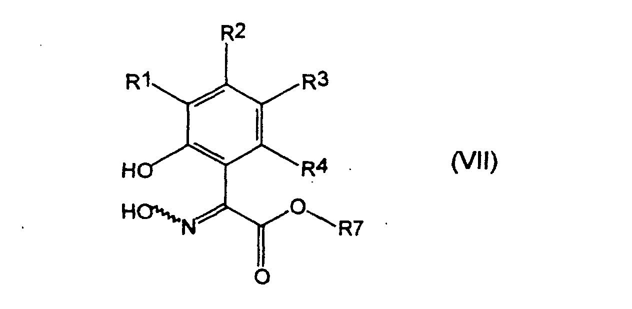 dans laquelle:
R¹, R², R³, R⁴ et R⁷ ont les significations indiquées ci-dessus,
et les composés ainsi obtenus de la formule (VII) sont mis à réagir selon l'étape de procédé 4), avec un agent d'alcoylation de la formule (VIII) :
R⁵ - X (VIII)
dans laquelle :
R⁵ a la signification indiquée ci-dessus, et
X représente un atome d'halogène ou le radical - O-SO₂-O-R⁵, où R⁵ a la signification indiquée ci-dessus,
le cas échéant en présence d'un agent de dilution et le cas échéant, en présence d'une base, ou
b) selon l'étape de procédé 5), sont mis à réagir avec une alcoxyamine de la formule (IX):
R⁵ - O - NH² (IX)
dans laquelle:
R⁵ a la signification indiquée ci-dessus,
ou un complexe d'addition d'acide de celle-ci, le cas échéant en présence d'un agent de dilution et le cas échéant, en présence d'un acide ou d'un accepteur d'acide,
ou **caractérisé en ce que**
B) selon l'étape de procédé 6), on fait réagir des composés de la formule (IV): 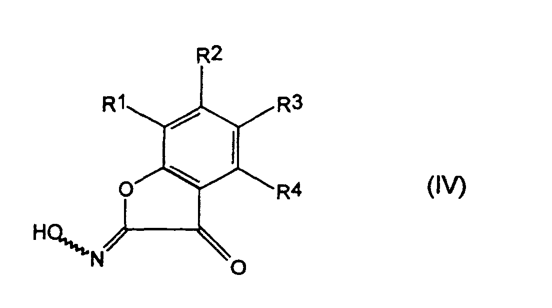 dans laquelle:
R¹, R², R³ et R⁴ ont les significations indiquées ci-dessus,
avec une alcoxyamine de la formule (IX) :
R⁵ - O - NH² (IX)
dans laquelle:
R⁵ a la signification indiquée ci-dessus,
ou un complexe d'addition d'acide de celle-ci, le cas échéant en présence d'un agent de dilution et le cas échéant, en présence d'un acide,
ou **caractérisé en ce que**
C) selon l'étape de procédé 7), on fait réagir des composés de la formule (IV): 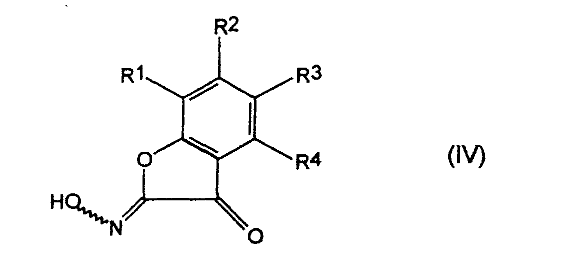 dans laquelle:
R¹, R², R³ et R⁴ ont les significations indiquées ci-dessus,
en présence d'un acide ou d'un échangeur d'ions acide, avec un alcool de la formule (V) :
R⁷ - OH (V)
dans laquelle:
R⁷ a la signification indiquée ci-dessus,
le cas échéant avec addition d'un sel d'hydroxylammonium, et les composés ainsi obtenus de la formule (VII) : 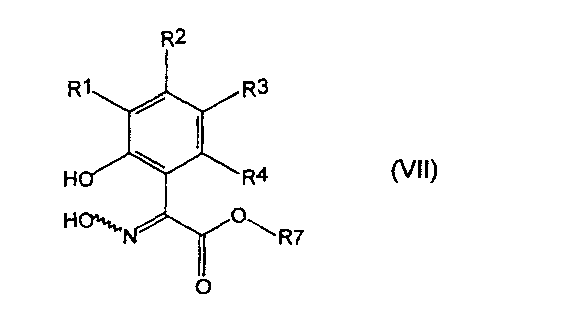
dans laquelle:
R¹, R², R³, R⁴ et R⁷ ont les significations indiquées ci-dessus,
sont mis à réagir selon l'étape de procédé 4),
ou **caractérisé en ce que**
D) selon l'étape de procédé 8), on fait réagir des composés de la formule (X) : 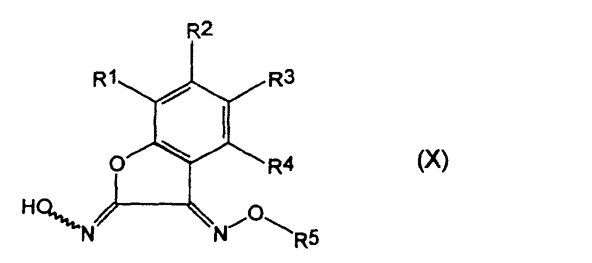 dans laquelle :
R¹, R², R³, R⁴ et R⁵ ont les significations indiquées ci-dessus,
en présence d'un acide ou d'un échangeur d'ions acide, avec un alcool de la formule (V) :
R⁷ - OH (V)
dans laquelle:
R⁷ a la signification indiquée ci-dessus,
le cas échéant en présence d'un composé carbonylé, qui lie le chlorure d'hydroxylammonium clivé lors de la réaction pour former un oxime,
et on fait réagir les composés obtenus selon A)-D) de la formule (II): 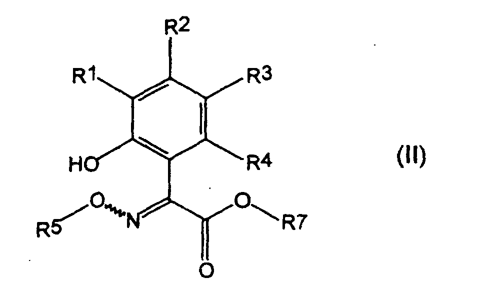
dans laquelle:
R¹, R², R³, R⁴ et R⁵ ont les significations indiquées ci-dessus, et
R⁷ représente un radical alcoyle substitué ou non substitué,
le cas échéant, sans isolement intermédiaire des composés de la formule (II) (procédé one-pot),
selon l'étape de procédé 1), avec une alcoylamine de la formule (III) :
R⁶ - NH₂ (III)
dans laquelle:
R⁶ a la signification indiquée ci-dessus,
le cas échéant en présence d'un agent de dilution.

2. Procédé suivant la revendication 1, **caractérisé en ce que** la partie 1Ab) du procédé (étapes de procédé 2, 5 et 1) est réalisée selon un procédé one-pot.

3. Procédé suivant la revendication 1, **caractérisé en ce que** l'on met en oeuvre comme agent de dilution le méthanol dans toutes les étapes de procédé.

4. Procédé suivant la revendication 1, **caractérisé en ce que** l'on utilise comme agent d'alcoylation, le chlorure de méthyle.

5. Procédé de préparation de composés de la formule (XI) : 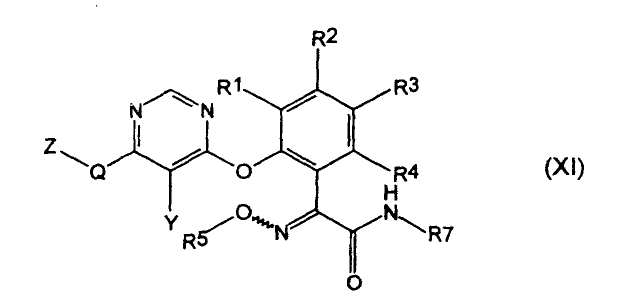 dans laquelle:
Z représente un radical cycloalcoyle, aryle ou hétérocyclyle non substitué ou substitué,
Q représente l'atome d'oxygène ou de soufre,
Y représente un atome d'halogène,
R¹, R², R³ et R⁴ sont identiques ou différents et représentent indépendamment l'un de l'autre, chaque fois l'atome d'hydrogène, un atome d'halogène, le radical cyano, nitro, un radical alcoyle, alcoxy, alcoylthio, alcoylsulfinyle ou alcoylsulfonyle chaque fois le cas échéant substitué par des atomes d'halogène,
R⁵ représente un radical alcoyle substitué ou non substitué, et
R⁷ représente un radical alcoyle substitué ou non substitué,
**caractérisé en ce que** l'on fait réagir les composés de la formule (I), préparés selon la revendication 1,
E) selon l'étape de procédé 9), avec des dérivés de la pyrimidine de la formule (XII) : 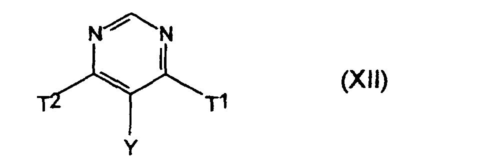 dans laquelle:
T¹ et T² sont identiques ou différents et représentent un atome d'halogène,
Y a la signification indiquée ci-dessus,
le cas échéant en présence d'un agent de dilution et le cas échéant, en présence d'une base, et les composés ainsi obtenus de la formule (XIII) : 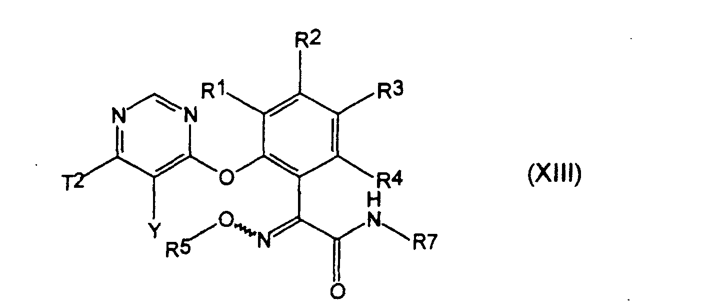
dans laquelle :
T², Y, R¹, R², R³, R⁴, R⁵ et R⁷ ont les significations indiquées ci-dessus, et
sont mis à réagir selon l'étape de procédé 10), avec un composé cyclique de la formule générale (XIV) :
Z - Q - H (XIV)
dans laquelle Z et Q ont les significations indiquées ci-dessus,
le cas échéant en présence d'un agent de dilution et le cas échéant, en présence d'un accepteur d'acide, et le cas échéant, en présence d'un catalyseur,
ou
F) selon l'étape de procédé 11), avec des composés de la formule (XV) : 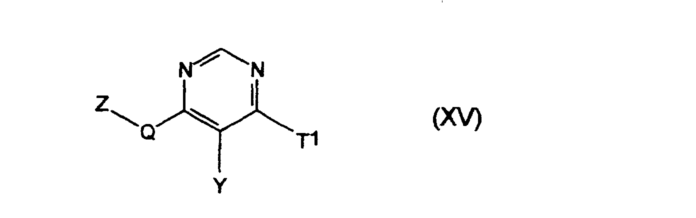 dans laquelle:
Z, Q, T¹ et Y ont les significations indiquées ci-dessus,
le cas échéant en présence d'un agent de dilution et le cas échéant, en présence d'une base.

6. Procédé suivant la revendication 5, **caractérisé en ce que** l'on fait réagir les composés de la formule I, préparés selon la revendication 1, selon l'étape de procédé 9), 10) ou 11) sans isolement des composés intermédiaires.

7. Composés de la formule (VII) : 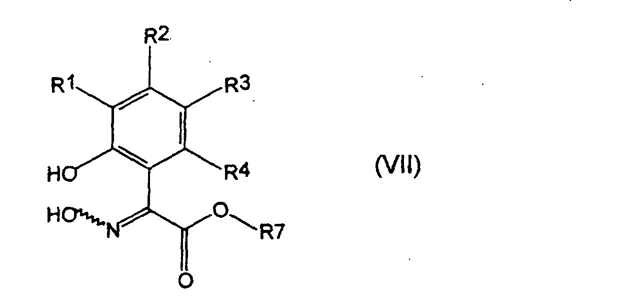 dans laquelle:
R¹, R², R³, R⁴ et R⁷ ont les significations indiquées à la revendication 1,
où
le composé (VII-a): 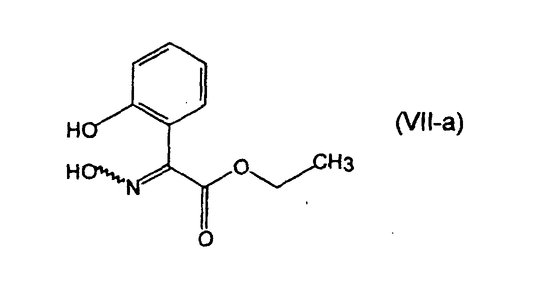 est exclu.

8. Utilisation des composés de la formule (VII) suivant la revendication 7, définis comme produits intermédiaire pour la préparation des composés de la formule (I), comme défini à la revendication 1.

9. Procédé de préparation de composés de la formule (VI): 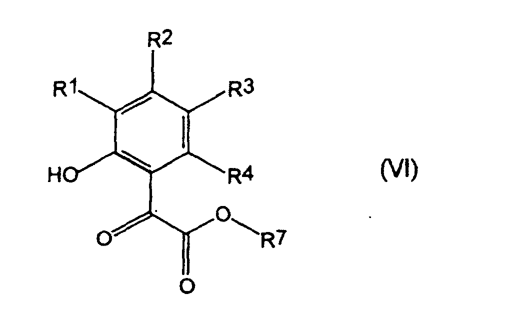 dans laquelle:
R¹, R², R³ et R⁴ sont identiques ou différents et représentent indépendamment l'un de l'autre, chaque fois l'atome. d'hydrogène, un atome d'halogène, le radical cyano, nitro, un radical alcoyle, alcoxy, alcoylthio, alcoylsulfinyle ou alcoylsulfonyle chaque fois le cas échéant substitué par des atomes d'halogène, et
R⁷ représente un radical alcoyle substitué ou non substitué,
**caractérisé en ce que** l'on fait réagir des composés de la formule (IV) : 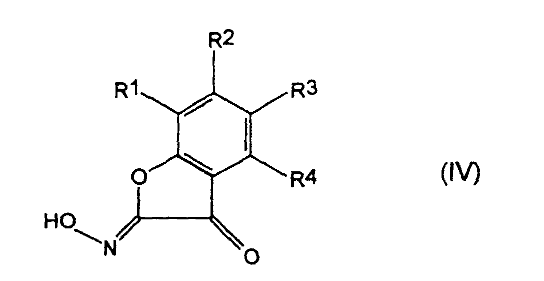 dans laquelle :
R¹, R², R³ et R⁴ ont les significations indiquées ci-dessus,
en présence d'un acide ou d'un échangeur d'ions acide, avec un alcool de la formule (V) :
R⁷ - OH (V)
dans laquelle:
R⁷ a la signification indiquée ci-dessus, et avec un composé carbonylé, qui lie le chlorure d'hydroxylammonium clivé lors de la réaction pour former un oxime.

10. Composé de la formule (XI-1, isomère E) :

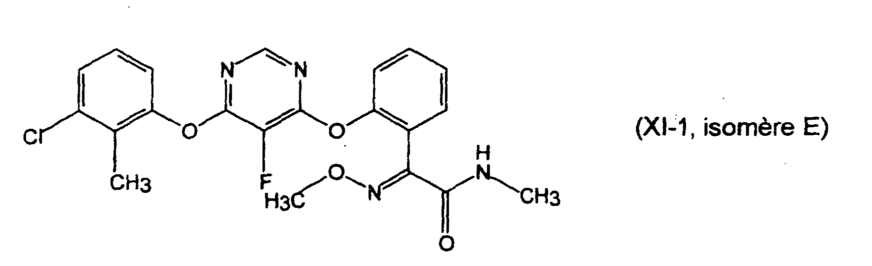

11. Composé de la formule (XI-1, isomère Z) :
